(19) Europäisches Patentamt
European Patent Office
Office européen des brevets

(11) EP 1 756 086 B1

(12) **EUROPEAN PATENT SPECIFICATION**

(45) Date of publication and mention of the grant of the patent:
**04.06.2008 Bulletin 2008/23**

(21) Application number: **05744719.5**

(22) Date of filing: **23.05.2005**

(51) Int Cl.:
***C07D 401/06*** (2006.01) ***C07D 401/14*** (2006.01)
***A61K 31/4439*** (2006.01) ***A61K 31/444*** (2006.01)
***A61P 25/22*** (2006.01)

(86) International application number:
**PCT/EP2005/005559**

(87) International publication number:
**WO 2005/118568 (15.12.2005 Gazette 2005/50)**

(54) **PYRIDIN-4-YL-ETHYNYL-IMIDAZOLES AND PYRAZOLES AS MGLU5 RECEPTOR ANTAGONISTS**

PYRIDIN-4-YL-ETHYNYLIMIDAZOLE UND -PYRAZOLE ALS ANTAGONISTEN DES MGLU5-REZEPTORS

PYRIDIN-4-YL-ETHYNYL-IMIDAZOLES ET PYRAZOLES, ANTAGONISTES DU RECEPTEUR MGLUR5

(84) Designated Contracting States:
**AT BE BG CH CY CZ DE DK EE ES FI FR GB GR HU IE IS IT LI LT LU MC NL PL PT RO SE SI SK TR**
Designated Extension States:
**HR LV YU**

(30) Priority: **01.06.2004 PCT/EP2004/005881**
**07.09.2004 EP 04021216**

(43) Date of publication of application:
**28.02.2007 Bulletin 2007/09**

(73) Proprietor: **F.HOFFMANN-LA ROCHE AG**
**4070 Basel (CH)**

(72) Inventors:
- **BUETTELMANN, Bernd**
**79650 Schopfheim (DE)**
- **CECCARELLI, Simona, Maria**
**CH-4057 Basel (CH)**
- **JAESCHKE, Georg**
**CH-4051 Basel (CH)**
- **KOLCZEWSKI, Sabine**
**79618 Rheinfelden (DE)**
- **PORTER, Richard, Hugh, Philip**
**CH-4153 Reinach (CH)**
- **VIEIRA, Eric**
**CH-4402 Frenkendorf (CH)**
- **SPURR, Paul**
**CH-4125 Riehen (CH)**

(74) Representative: **Braun, Axel**
**F.Hoffmann-La Roche AG**
**Patent Department (PLP),**
**124 Grenzacherstrasse**
**4070 Basel (CH)**

(56) References cited:
**WO-A-01/16121** **WO-A-02/46166**
**WO-A-20/04080998** **WO-A-20/05003117**
**WO-A-20/05023795**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 756 086 B1

Printed by Jouve, 75001 PARIS (FR)

## Description

The present invention relates to diazole derivatives of the general formula

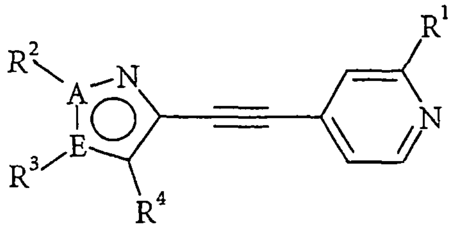

(I)

wherein
one of A or E is N and the other is C;

| | |
|---|---|
| $R^1$ | is halogen or cyano; |
| $R^2$ | is lower alkyl; |
| $R^3$ | is aryl or heteroaryl, which are optionally substituted by: one, two or three substituents, selected from the group consisting of halogen, lower alkyl, lower alkoxy, cycloalkyl, lower haloalkyl, lower haloalkoxy, cyano, NR'R" or by 1-morpholinyl, or by<br>1-pyrrolidinyl, optionally substituted by $(CH_2)_{0,1}OR$, or by<br>piperidinyl, optionally substituted by $(CH_2)_{0,1}OR$, or by<br>1,1-dioxo-thiomorpholinyl or by<br>piperazinyl, optionally substituted by lower alkyl or $(CH_2)_{0,1}$-cycloalkyl; |
| R | is hydrogen, lower alkyl or $(CH_2)_{0,1}$-cycloalkyl; |
| R', R" | are independently from each other hydrogen, lower alkyl, $(CH_2)_{0,1}$-cycloalkyl or $(CH_2)_nOR$; |
| n | is 1 or 2; |
| $R^4$ | is $CHF_2$, $CF_3$, or C(O)H, $CH_2R^5$ wherein $R^5$ is hydrogen, OH, $C_1$-$C_6$-alkyl or $C_3$-$C_{12}$-cycloalkyl; |

as well as to pharmaceutically acceptable salts thereof.

It has now surprisingly been found that the compounds of general formula I are metabotropic glutamate receptor antagonists. Compounds of formula I are distinguished by having valuable therapeutic properties. They can be used in the treatment or prevention of mGluR5 receptor mediated disorders.

In the central nervous system (CNS) the transmission of stimuli takes place by the interaction of a neurotransmitter, which is sent out by a neuron, with a neuroreceptor.

Glutamate is the major excitatory neurotransmitter in the brain and plays a unique role in a variety of central nervous system (CNS) functions. The glutamate-dependent stimulus receptors are divided into two main groups. The first main group, namely the ionotropic receptors, forms ligand-controlled ion channels. The metabotropic glutamate receptors (mGluR) belong to the second main group and, furthermore, belong to the family of G-protein coupled receptors.

At present, eight different members of these mGluR are known and of these some even have sub-types. According to their sequence homology, signal transduction mechanisms and agonist selectivity, these eight receptors can be sub-divided into three sub-groups:

mGluR1 and mGluR5 belong to group I, mGluR2 and mGluR3 belong to group II and mGluR4, mGluR6, mGluR7 and mGluR8 belong to group III.

Ligands of metabotropic glutamate receptors belonging to the first group can be used for the treatment or prevention of acute and/or chronic neurological disorders such as psychosis, epilepsy, schizophrenia, Alzheimer's disease, cognitive disorders and memory deficits, as well as chronic and acute pain.

Other treatable indications in this connection are restricted brain function caused by bypass operations or transplants, poor blood supply to the brain, spinal cord injuries, head injuries, hypoxia caused by pregnancy, cardiac arrest and hypoglycaemia. Further treatable indications are ischemia, Huntington's chorea, amyotrophic lateral sclerosis (ALS), dementia caused by AIDS, eye injuries, retinopathy, idiopathic parkinsonism or parkinsonism caused by medicaments as well as conditions which lead to glutamate-deficiency functions, such as e.g. muscle spasms, convulsions, migraine, urinary incontinence, nicotine addiction, opiate addiction, anxiety, vomiting, dyskinesia and depressions.

Disorders mediated full Or in part by mGluR5 are for example acute, traumatic and chronic degenerative

processes of the nervous system, such as Alzheimer's disease, senile dementia, Parkinson's disease, Huntington's chorea, amyotrophic lateral sclerosis and multiple sclerosis, psychiatric diseases such as schizophrenia and anxiety, depression, pain and drug dependency (Expert Opin. Ther. Patents (2002),12, (12)).

Selective mGIuR5 antagonists are especially useful for the treatment of anxiety and pain.

WO 02/46166 discloses phenyl-ethynyl-imidazoles and pyrazoles as mGluR5 anatgonists. The phenyl-ethynyl-imidazoles according to the present invention differ from the compounds of WO 02/46166 through the pyridine-4-yl moiety and the meaning of R3. Similarly, the phenyl-ethynyl-pyrazoles differ from the compounds of WO 02/46166 through the pyridine-4-yl moiety and the pyrazole substituents.

WO 01/16121 relates to mGluR modulators of the formula B-L-A, wherein B is inter alia substituted pyridyl; L is alynylene and A includes imidazolyl and pyrazolyl.

The invention relates to compounds of formula I and their pharmaceutically acceptable salts, to the above-mentioned compounds as pharmaceutically active substances and their production.

The invention also relates to a process for preparing a compound according to general formula I following the general procedures as outlined above for compounds of formula I.

Moreover the invention relates also to medicaments containing one or more compounds of the present invention and pharmaceutically acceptable excipients for the treatment and prevention of mGluR5 receptor mediated disorders, such as acute and/or chronic neurological disorders, in particular anxiety and chronic or acute pain, protection against liver damage or failure whether drug or disease induced.

The invention also relates to the use of a compound in accordance with the present invention as well as its pharmaceutically acceptable salt for the manufacture of medicaments for the treatment and prevention of mGluR5 receptor mediated disorders as outlined above.

The following definitions of general terms used in the present description apply irrespective of whether the terms in question appear alone or in combination.

The term "lower alkyl" used in the present description denotes straight-chain or branched saturated hydrocarbon residues with 1 to 6 carbon atoms, preferably with 1 to 4 carbon atoms, such as methyl, ethyl, n-propyl, i-propyl, n-butyl, t-bntyl and the like.

The term "lower alkoxy" denotes a lower alkyl residue in the sense of the foregoing definition bound via an oxygen atom. Examples of "lower alkoxy" residues include methoxy, ethoxy, isopropoxy and the like.

The tem "halogen" denotes fluorine, chlorine, bromine and iodine.

"lower haloalkoxy" denotes lower alkoxy group as defined above which is substituted by one or more halogen. Examples of lower haloalkoxy include but are not limited to methoxy or ethoxy, substituted by one or more Cl, F, Br or I atom(s) as well as those groups specifically illustrated by the examples herein below. Prefered lower haloalkoxy are difluoro- or trifluoro-methoxy or ethoxy.

"lower haloalkyl" denotes a lower alkyl group as defined above which is substituted by one or more halogen. Examples of lower haloalkyl include but are not limited to methyl, ethyl, propyl, isopropyl, isobutyl, sec-butyl, tert-butyl, pentyl or n-hexyl substituted by one or more Cl, F, Br or I atom(s) as well as those groups specifically illustrated by the examples herein below. Prefered lower haloalkyl are difluoro- or trifluoro-methyl or ethyl.

"Aryl" represents an aromatic carbocyclic group consisting of one individual ring, or one or more fused rings in which at least one ring is aromatic in nature. Preferred aryl group is phenyl.

The term "heteroaryl" refers to an aromatic 5- or 6-membered ring containing one or more heteroatoms selected from nitrogen, oxygen or sulphur. Preferred are those heteroaryl groups selected from nitrogen. Examples of such heteroaryl groups are pyridinyl, pyrazinyl, pyrimidinyl or pyridazinyl.

The term "cycloalkyl" denotes a saturated carbocyclic group, containing 3-12 carbon atoms, preferably 3-6 carbon atoms.

The term "pharmaceutically acceptable salt" refers to any salt derived from an inorganic or organic acid or base. Such salts include: acid addition salts formed with inorganic acids such as hydrochloric acid, hydrobromic acid, sulfuric acid, nitric acid, phosphoric acid; or formed with organic acids such as acetic acid, benzenesulfonic acid, benzoic, camphorsulfonic acid, citric acid, ethanesulfonic acid, fumaric acid, glucoheptonic acid, gluconic acid, glutamic acid, glycolic acid, hydroxynaphtoic acid, 2-hydroxyethanesulfonic acid, lactic acid, maleic acid, malic acid, malonic acid, mandelic acid, methanesulfonic acid, muconic acid, 2-naphthalenesulfonic acid, propionic acid, salicylic acid, succinic acid, tartaric acid, p-toluenesulfonic acid or trimethylacetic acid.

Preferred compounds of formula I are those compounds of formulae Ia and Ib:

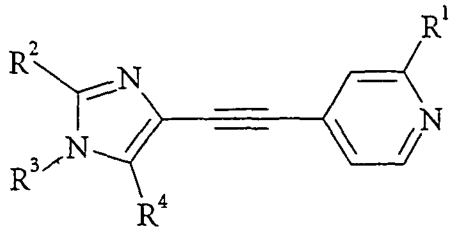

(Ia)

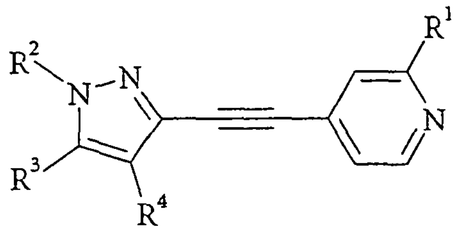

(Ib)

wherein $R^1$, $R^2$, $R^3$ and $R^4$ are as defined herein above.

In the compounds of formulae I, Ia or Ib, according to the invention, preferred $R^1$ is halogen and preferably chloro or cyano.

Preferred $R^2$ is methyl or i-propyl.

Preferred $R^3$ is selected from phenyl, pyridinyl, pyrazinyl, pyrimidinyl or pyridazinyl which may be substituted by one or more chloro, fluoro, lower alkyl, lower alkoxy, cyano, lower haloalkyl, lower haloalkoxy or cycloalkyl.

Preferred $R^4$ is lower alkyl, $CHF_2$ or $CH_2OH$ and preferably methyl.

Preferred compounds are those compounds of formulae Ia and Ib wherein:

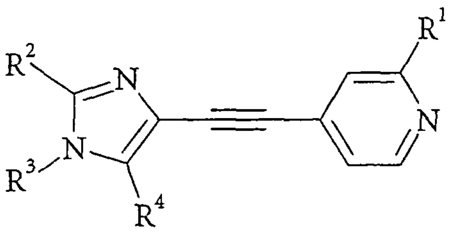

(Ia)

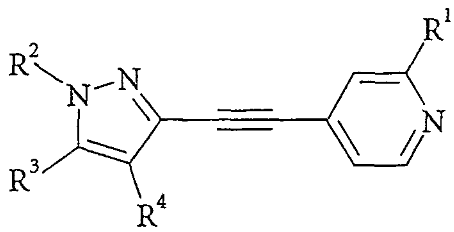

(Ib)

$R^1$ is halogen and preferably chloro or cyano;

$R^2$ is methyl or i-propyl;

$R^3$ is selected from phenyl, pyridinyl, pyrazinyl, pyrimidinyl or pyridazinyl which may be substituted by one or more chloro, fluoro, lower alkyl, lower alkoxy, cyano, lower haloalkyl, lower haloalkoxy or cycloalkyl; and

$R^4$ is $CHF_2$, $CH_2R^5$ wherein $R^5$ is hydrogen, OH or $C_1$-$C_6$-alkyl and preferably $R^4$ is methyl;

and pharmaceutically acceptable salts thereof.

Preferred are those compounds of formula Ia, wherein, $R^3$ is unsubstituted or substituted heteroaryl, wherein the substitution is selected from chloro, fluoro, $CF_3$, and lower alkyl, for example the following compounds:

2-[4-(2-Chloro-pyridin-4-ylethynyl)-2,5-dimethyl-1H-imidazol-1-yl]-5-methyl-pyridine;
2-Chloro-5-[4-(2-Chloro-pyridin-4-ylethynyl)-2,5-dimethyl-1H-imidazol-1-yl]-pyridine;
2-[4-(2-Chloro-pyridin-4-ylethynyl)-2,5-dimethyl-1H-imidazol-1-yl]-6-methyl-4-trifluoromethyl-pyridine;
2-[4-(2-Chloro-pyridin-4-ylethynyl)-2,5-dimethyl-1H-imidazol-1-yl]-pyrazine;
2-[4-(2-Chloro-pyridin-4-ylethynyl)-2,5-dimethyl-1H-hnidazol-1-yl]-6-methyl-pyridine;
2-[4-(2-Chloro-pyridin-4-ylethynyl)-2,5-dimethyl-1H-imidazol-1-yl]-6-(trifluoromethyl)-pyridine; and
3-[4-(2-Chloro-pyridin-4-ylethynyl)-2,5-dimethyl-1H-imidazol-1-yl]-5-fluoro-pyridine.

Especially preferred are further those compounds of formula Ia, wherein, $R^3$ is aryl, substituted by one or more chloro, fluoro, $CF_3$, and lower alkyl, lower alkoxy, $CF_3O$, 1-morpholinyl, for example the following compounds:

2-Chloro-4-[1-(4-fluoro-phenyl)-2,5-dimethyl-1H-imidazol-4-ylethynyl]-pyridine;
2-Chloro-4-[1-(2,4-difluoro-phenyl)-2,5-dimethyl-1H-imidazol-4-ylethynyl]-pyridine;
2-Chloro-4-[1-(3,5-difluoro-phenyl)-2,5-dimethyl-1H-imidazol-4-ylethynyl]-pyridine;
2-Chloro-4-[1-(4-fluoro-2-methyl-phenyl)-2,5-dimethyl-1H-imidazol-4-ylethynyl]-pyridine;

2-Chloro-4-[1-(4-fluoro-3-methyl-phenyl)-2,5-dimethyl-1H-imidazol-4-ylethynyl]-pyridine;
2-Chloro-4-(2,5-dimethyl-1-p-tolyl-1H-imidazol-4-ylethynyl)-pyridine;
2-Chloro-4-[1-(3-chloro-4-methyl-phenyl)-2,5-dimethyl-1H-imidazol-4-ylethynyl]-pyridine;
2-Chloro-4-[1-(3-fluoro-4-methoxy-phenyl)-2,5-dimethyl-1H-imidazol-4-ylethynyl]-pyridine;
2-Chloro-4-[1-(4-methoxy-phenyl)-2,5-dimethyl-1H-imidazol-4-ylethynyl]-pyridine;
2-Chloro-4-[2,5-dimethyl-1-(4-trifluoromethoxy-phenyl)-1H-imidazol-4-ylethynyl]-pyridine;
2-Chloro-4-[2,5-dimethyl-1-(3-trifluoromethoxy-phenyl)-1H-imidazol-4-ylethynyl]-pyridine;
2-Chloro-4-[2,5-dimethyl-1-(4-trifluoromethyl-phenyl)-1H-imidazol-4-ylethynyl]-pyridine;
2-Chloro-4-[2,5-dimethyl-1-(3-methyl-4-trifluoromethoxy-phenyl)-1H-imidazol-4-ylethynyl] -pyridine;
2-Chloro-4-[1-(4-chloro-phenyl)-2,5-dimethyl-1H-imidazol-4-ylethynyl]-pyridine;
2-Chloro-4-[1-(3-cbloro-2-fluoro-phenyl)-2,5-dimethyl-1H-imidazol-4-ylethynyl]-pyridine;
2-Chloro-4-[2,5-dimethyl-1-(3-trifluoromethyl-phenyl)-1H-infdazol-4-ylethynyl]-pyridine;
2-Chloro-4-[1-(3-chloro-4-fluoro-phenyl)-2,5-dimethyl-1H-imidazol-4-ylethynyl]-pyridine;
2-Chloro-4-[2,5-dimethyl-1-(2-methyl-4-trifluoromethoxy-phenyl)-1H-imidazol-4-ylethynyl] -pyridine;
2-Chloro-4-[5-difluoromethyl-1-(4-fluoro-phenyl)-2-methyl-1H-imidazol-4-ylethynyl]-pyridine;
[5-(2-Chloro-pyridin-4-ylethynyl)-3-(4-fluoro-phenyl)-2-methyl-3H-imidazol-4-yl]-methanol;
2-Chloro-4-[1-(4-methoxy-3-trifluoromethyl-phenyl)-2,5-dimethyl-1H-imidazol-4-ylethynyl]-pyridine;
2-Chloro-4-[1-(3,5-difluoro-4-methoxy-phenyl)-2,5-dimethyl-1H-imidazol-4-ylethynyl]-pyridine;
2-Chloro-4-[1-(4-methoxy-3-trifluoromethoxy-phenyl)-2,5-dimethyl-1H-imidazol-4-ylethynyl] -pyridine;
2-Chloro-4-[1-(3-methoxy-4-trifluoromethoxy-phenyl)-2,5-dimethyl-1H-imidazol-4-ylethynyl]-pyridine;
4-{3-[4-(2-Chloro-pyridin-4-ylethynyl)-2,5-dimethyl-imidazol-1-yl]-5-fluoro-phenyl}-morpholine;
2-Chloro-4-[1-(4-fluoro-2-trifluoromethoxy-phenyl)-2,5-dimethyl-1H-imidazol-4-ylethynyl]-pyridine;
2-Chloro-4-[1-(2-fluoro-4-trifluoromethoxy-phenyl)-2,5-dimethyl-1H-imidazol-4-ylethynyl]-pyridine;
2-Chloro-4-[2,5-dimethyl-1-(4-methyl-3-trifluoromethyl-phenyl)-1H-imidazol-4-ylethynyl] -pyridine;
2-Chloro-4-[2,5-dimethyl-1-(3-methyl-4-trifluoromethyl-phenyl)-1H-imidazol-4-ylethynyl] -pyridine;
2-Chloro-4-[2,5-dimethyl-1-(3-methyl-5-trifluoromethyl-phenyl)-1H-imidazol-4-ylethynyl] -pyridine;
2-Chloro-4-[1-(3-methoxy-5-trifluoromethyl-phenyl)-2,5-dimethyl-1H-imidazol-4-ylethynyl] -pyridine;
2-Chloro-4- [1-(3-methoxy-4-trifluoromethyl-phenyl)-2,5-dimethyl-1H-imidazol-4-ylethynyl]-pyridine;
2-Chloro-4- [1-(3,5-dichloro-phenyl)-2,5-dimethyl-1H-imidazol-4-ylethynyl]-pyridine;
2-Chloro-4-[1-(3-chloro-5-methyl-phenyl)-2,5-dimethyl-1H-imidazol-4-ylethynyl]-pyridine;
2-Chloro-4- [1-(3-fluoro-5-methyl-phenyl)-2,5-dimethyl-1H-imidazol-4-ylethynyl]-pyridine;
2-Chloro-4- [1-(3-chloro-5-methoxy-phenyl)-2,5-dimethyl-1H-imidazol-4-ylethynyl]-pyridine; and
2-Chloro-4- [1-(3-fluoro-5-methoxy-phenyl)-2,5-dimethyl-1H-imidazol-4-ylethynyl]-pyridine.

Still other preferred are further those compounds of formula Ib, wherein $R^3$ is aryl, substituted by one or more fluoro, especially the following compound: 2-Chloro-4-[5-(4-fluoro-phenyl)-1,4-dimethyl-1H-pyrazol-3-ylethynyl]-pyridine.

The compounds of formula Ia of the invention may be prepared according to various processes.
In an embodiment, the process of the invention comprises the following steps of reacting a compound of formula II

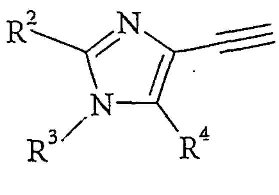

(II)

with a compound of formula III

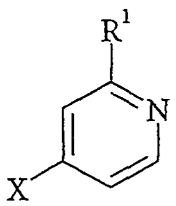

(III)

in order to obtain the compound of formula Ia;
wherein $R^1$, $R^2$, $R^3$ and $R^4$ are as defined above and X is halogen, and if desired, converting the compounds obtained into pharmaceutically acceptable acid addition salts. This process is described in more detail in scheme 1 and general procedure 1.

In another embodiment, the compounds of formula Ia may be prepared according to the following process of the invention which comprises the step of reacting a compound of formula IV

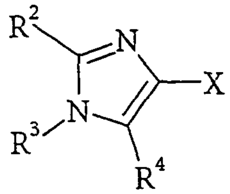

(IV)

with a compound of formula V

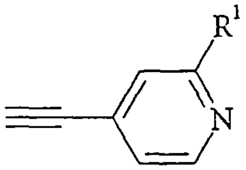

(V)

in order to obtain the compound of formula Ia;
wherein $R^1$, $R^2$, $R^3$ and $R^4$ are as defined above and X is halogen, and if desired, converting the compounds obtained into pharmaceutically acceptable acid addition salts. This process is described in more detail in scheme 2 and general procedure 2.

In still another embodiment, the compounds of formula Ia may be prepared according to the following process of the invention which comprises the step of reacting a compound of formula Ic

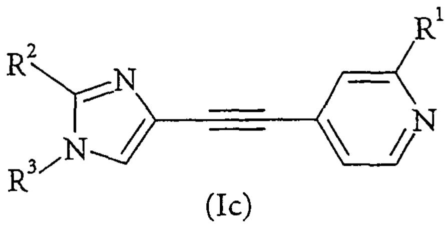

(Ic)

with a compound of formula VI

$R^4$-X (VI)

in order to obtain the compound of formula Ia;
wherein $R^1$, $R^2$, $R^3$ and $R^4$ are as defined above and X is halogen, and if desired, converting the compounds obtained into pharmaceutically acceptable acid addition salts. This process is described in more detail in scheme 3 and general procedure 3.

The compounds of formula Ib may be prepared according to the following process of the invention which comprises the step of reacting a compound of formula XXVI

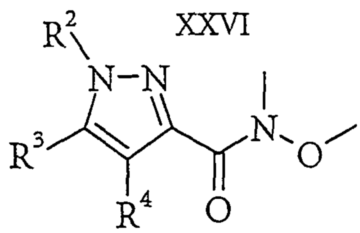

XXVI

with a compound of formula XXVII

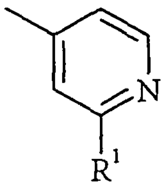

XXVII

in order to obtain a compound of formula XXVIII

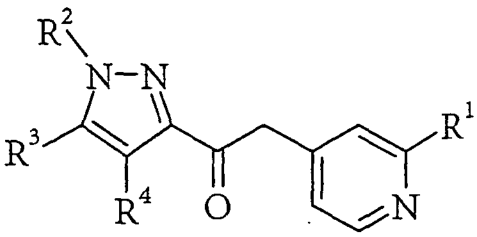

XXVIII

and convert the compound of formula XXVIII into the compound of formula Ib;
wherein $R^1$, $R^2$, $R^3$ and $R^4$ are as defined above, and if desired, converting the compounds obtained into pharmaceutically acceptable acid addition salts. This process is described in more detail in scheme 4 and general procedure 4.

The various processes of the invention are described in more detail in the following schemes with the following general procedures:

Scheme 1

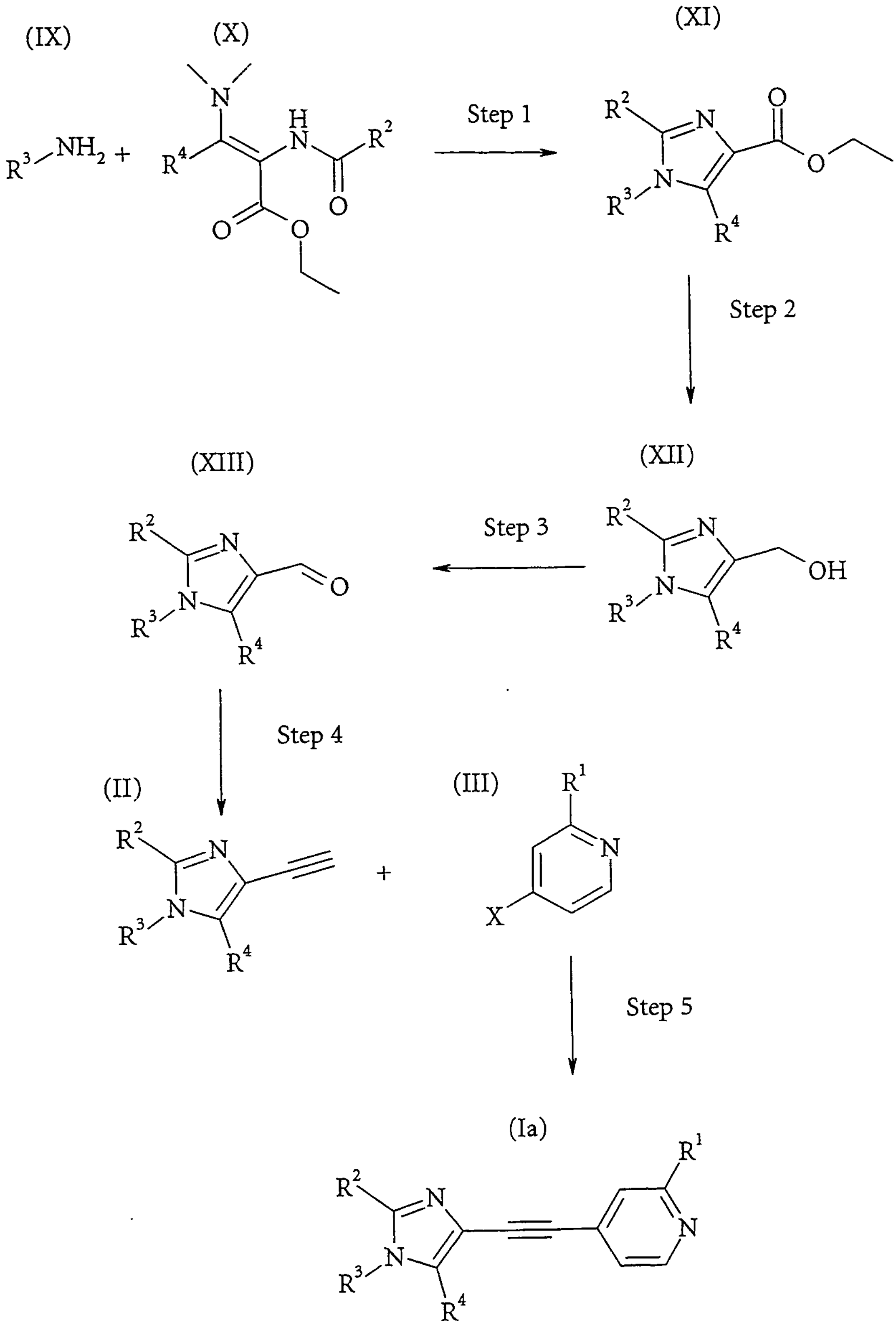


(IX) (X) (XI) (XII) (XIII) (II) (III) (Ia)

General procedure 1

In the scheme 1, $R^1$, $R^2$, $R^3$ and $R^4$ are as defined hereinabove.

Step 1: compound of formula XI

The compound of formula X, which preparation is disclosed herein in the part synthesis of intermediates (see Example A), and compound IX are reacted at room temperature in the appropriate solvent (e.g. acetic acid). The crude product is isolated and purified by conventional methods.

Step 2: compound of formula XII

The compound of formula XI is dissolved in the appropriate solvent (e.g.dry THF) and cooled. The appropriate reducing agent is added (e.g. Lithium aluminum hydride).

Step 3: compound of formula XIII

The compound of formula XII is dissolved the appropriate solvent (e.g. dichloromethane) and the appropriate oxidizing agent is added (e.g. Mangan(IV)oxid).

Step 4: compound of formula II

The compound XIII is reacted with (1-Diazo-2-oxo-propyl)-phosphonic acid dimethyl ester. The crude product is isolated and purified by conventional methods.

Step 5: compound of formula Ia

The compound of formula II is reacted with the compound of formula III with the appropriate catalysts (e.g. Triphenylphosphine, bis(triphenylphosphine)palladium(II)chloride and Copper(I)iodide). The crude product is isolated and purified by conventional methods.

Scheme 1 and general procedure 1 are further illustrated in the section examples herein.

Scheme 2

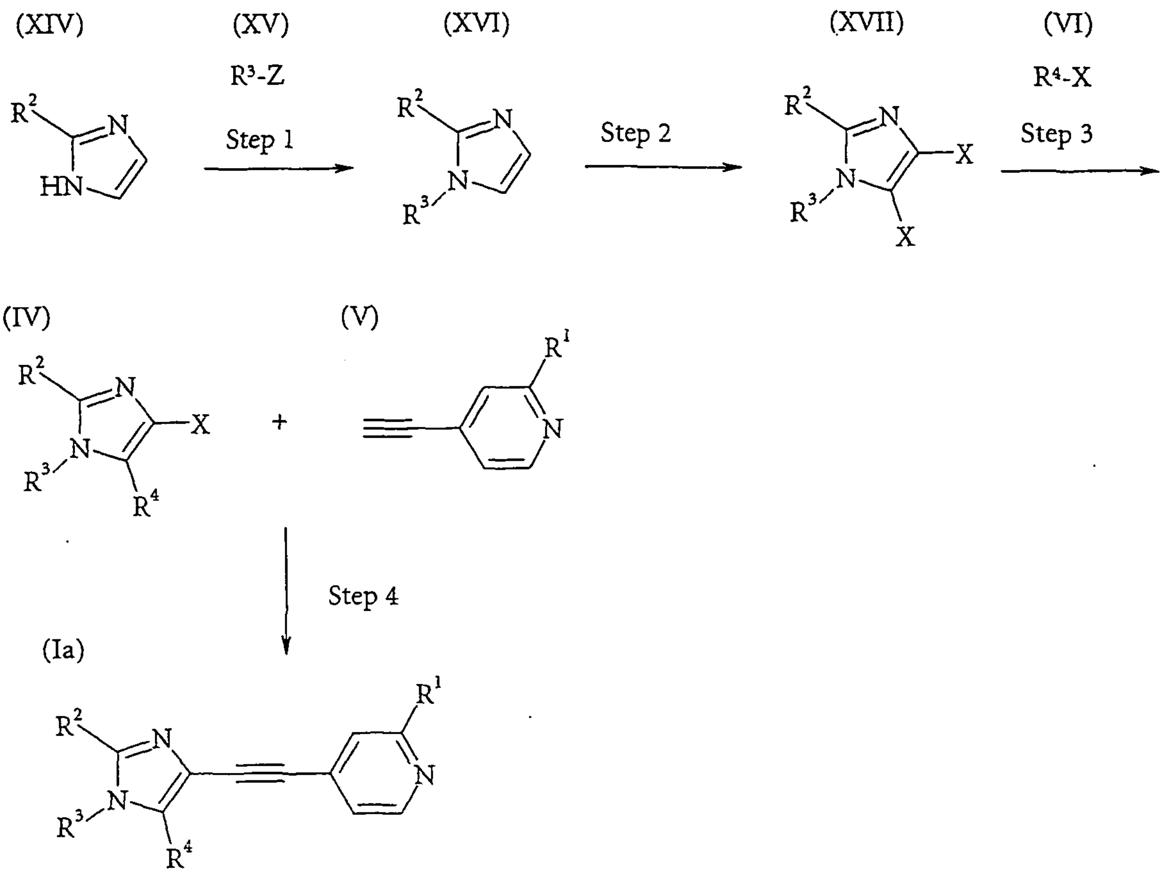


General procedure 2

In the scheme 2, $R^1$, $R^2$, $R^3$ and $R^4$ are as defined hereinabove.

Step 1: compound of formula XVI

The compound of formula XIV is reacted with a compound of formula XV (Z is preferably $B(OH)_2$) with the appropriate catalyst (e.g. $[Cu(OH)TMEDA]_2Cl_2$ (1.13 g, 2 mmol)).

Step 2: compound of formula XVII

The compound of formula XVI is reacted with the appropriate halogen X (e.g. Iodine in Iodic acid, acetic acid, and concentrated sulfuric acid in water with carbon tetrachloride) and stirred over night. The crude product is isolated and purified by conventional methods.

Step 3: compound of formula IV

The compound of formula XVII is reacted with the compound of formula VI wherein X is halogen (e.g. iodomethane). The crude product is purified by conventional methods.

Step 4: compound of formula Ia

Solution 1: the compound of formula IV, which preparation is disclosed herein in the part synthesis of intermediates (see Example C) and the compound of formula V are mixed under inert gas (e.g. argon).

Solution 2: The appropriate catalyst mixture is prepared under inert gas (e.g. triphenylphosphine, bis(triphenylphosphine)-palladium(II)chloride, copper(I)iodide and triethyl amine in THF).

Solutions 1 and 2 are mixed under heating (e.g. 40°C) and stirred. The crude product is purified by conventional methods.

Scheme 2 and general procedure 2 are further illustrated in the section examples herein.

Scheme 3

(Ic) (VI) (Ia)

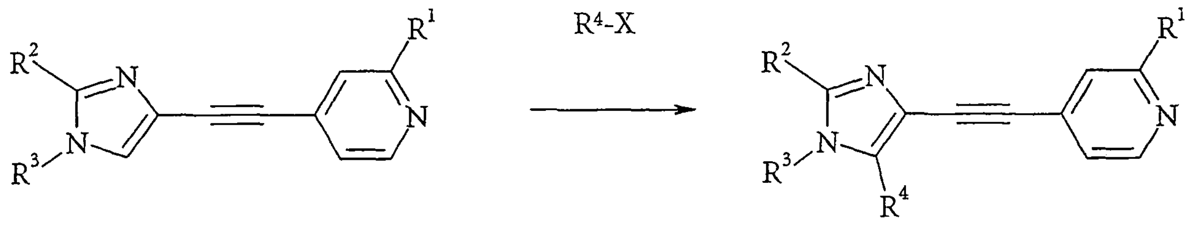


General procedure 3

In the scheme 3, $R^1$, $R^2$, $R^3$ and $R^4$ are as defined hereinabove.
The compound of formula Ic is reacted with the compound of formula VI wherein X is halogen (e.g. iodomethane). The crude product is purified by conventional methods.

Scheme 3 and general procedure 3 are further illustrated in the section examples herein.

Scheme 4

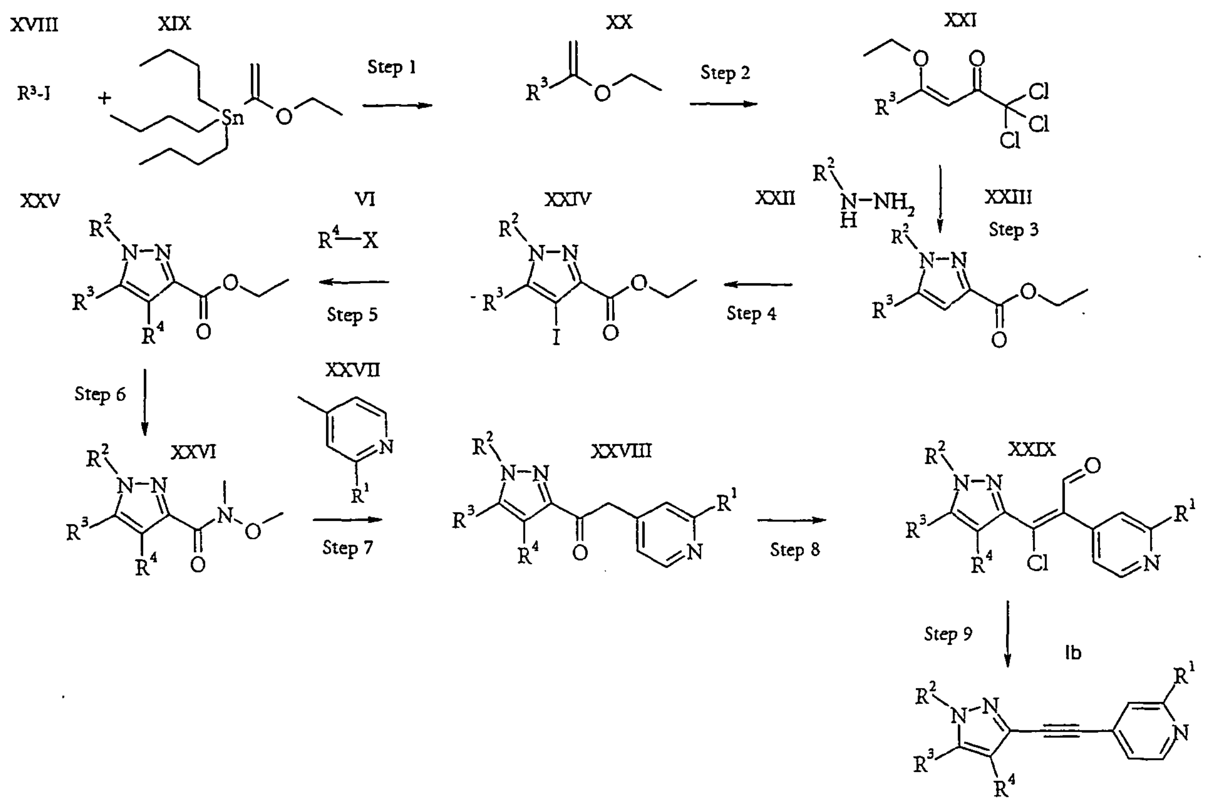

General procedure 4

In the scheme 4, $R^1$, $R^2$, $R^3$ and $R^4$ are as defined hereinabove.

Step 1: compound of formula XX

A solution of the compound of formula XVIII and of the compound of formula XIX are reacted with the appropriate catalyst (e.g. 1-Ethoxyvinyltributyltin and Tetrakis(triphenylphosphine)Palladium in 130 ml of Toluene) The crude product is used directly in the next step.

Step 2: compound of formula XXI

A solution of the compound of formula XX (e.g. in pyridine) is added to Trichloroacetyl chloride. The product of formula XXI is obtained by conventional work up.

Step 3: compound of formula XXIII

A solution of the compound of formula XXI (e.g. in ethanol) is added to a solution of the compound of formula XII. The compound of formula XXIII is obtained by conventional work up.

Step 4: compound of formula XXIV

To a well stirred solution of the compound of formula XXIII (e.g. in Acetonitrile) are added Iodine and Cerium ammonium nitrate. The compound of formula XXIV is obtained by conventional work up.

Step 5: compound of formula XXV

To a solution of compound of formula XXIV (e.g. in dry THF) is added a solution of n-Butyllithium in THF. The compound of formula VI is then added (X is halogen). The product of formula XXV is obtained by conventional work up.

Step 6: compound of formula XXVI

To a suspension of N,O-Dimethylhydroxylamine ethyl ester (e.g.in dry methylene chloride) is added a solution of Trimethylaluminium (e.g. in Heptane). Then a solution of the compound of formula XXV is added (e.g. in dry methylene chloride). The product of formula XXVI is obtained by conventional work up.

Step 7: compound of formula XXVIII

A solution of sodium-bis(trimethylsilyl) amide (e.g. in THF) is added to the compound of formula XXVII (e.g. in dry THF). Then a solution of the compound of formula XXVI (e.g. in dry THF) is added. The product of formula XXVIII is obtained by conventional work up.

Step 8: compound of formula XXIX

To dry Methylene chloride is added Chloromethylene-dimethylimidium chloride. Then a solution of compound of formula XXVIII (e.g. in dry methylene chloride) is added. The product of formula XXVIII is obtained by conventional work up.

Step 9: compound of formula Ib

To Potassium tert-butylate (e.g. in THF and water is added a solution of the compound of formula XXIX (e.g. in THF). The desired product of formula Ib is obtained by conventional work up.
Scheme 4 and general procedure 4 are further illustrated in the section examples herein.

Scheme 5

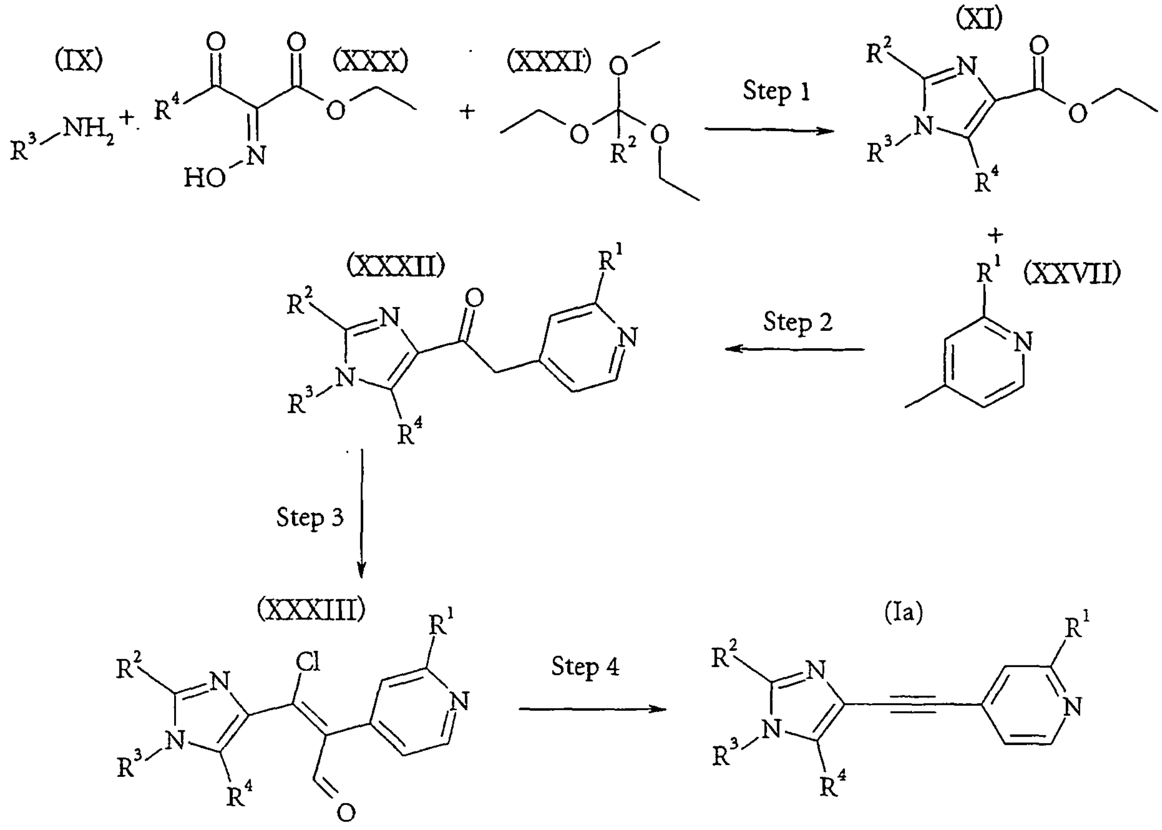


General procedure 5

In the scheme 5, $R^1$, $R^2$, $R^3$ and $R^4$ are as defined hereinabove.

Step 1: compound of formula XI

The compound of formula XXX, which preparation is disclosed herein in the part synthesis of intermediates (see Example C), and compound IX are reacted at room temperature or higher temperature in the appropriate solvent (e.g. toluene). The crude product is concentrated and reacted with a compound of formula XXXI under hydrogen atmosphere in the presence of palladium to form a compound of formula XI which is isolated and purified by conventional methods.

Step 2: compound of formula XXXII

A solution of sodium-bis(trimethylsilyl)amide (e.g. in THF) is added to the compound of formula XXVII (e.g. in dry THF). Then a solution of the compound of formula XI (e.g. in dry THF) is added. The product of formula XXXII is obtained by conventional work up.

Step 3: compound of formula XXXIII

To dry methylene chloride is added chloromethylene-dimethylimidium chloride. Then a solution of compound of formula XXXII (e.g. in dry methylene chloride) is added. The product of formula XXXIII is obtained by conventional work up.

Step 4: compound of formula Ib

To Potassium tert-butylate (e.g. in THF and water is added a solution of the compound of formula XXXIII (e.g. in THF). The desired product of formula Ib is obtained by conventional work up.

Scheme 5 and general procedure 5 are further illustrated in the section examples herein.

Pharmaceutically acceptable salts of compounds of formulae I, Ia and Ib can be manufactured readily according to methods known per se and taking into consideration the nature of the compound to be converted into a salt. Inorganic or organic acids such as, for example, hydrochloric acid, hydrobromic acid, sulphuric acid, nitric acid, phosphoric acid or citric acid, formic acid, fumaric acid, maleic acid, acetic acid, succinic acid, tartaric acid, methanesulphonic acid, p-toluenesulphonic acid and the like are suitable for the formation of pharmaceutically acceptable salts of basic compounds of formula I. Compounds which contain the alkali metals or alkaline earth metals, for example sodium, potassium, calcium, magnesium or the like, basic amines or basic amino acids are suitable for the formation of pharmaceutically acceptable salts of acidic compounds.

The compounds of formulae I, Ia and Ib and their pharmaceutically acceptable salts are, as already mentioned above, metabotropic glutamate receptor antagonists and can be used for the treatment or prevention of mGluR5 receptor mediated disorders, such as acute and/or chronic neurological disorders, cognitive disorders and memory deficits, as well as acute and chronic pain. Treatable neurological disorders are for instance epilepsy, schizophrenia, anxiety, acute, traumatic or chronic degenerative processes of the nervous system, such as Alzheimer's disease, senile dementia, Huntington's chorea, ALS, multiple sclerosis, dementia caused by AIDS, eye injuries, retinopathy, idiopathic parkinsonism or parkinsonism caused by medicaments as well as conditions which lead to glutamate-deficient functions, such as e.g. muscle spasms, convulsions, migraine, urinary incontinence, ethanol addiction, nicotine addiction, psychoses, opiate addiction, anxiety, vomiting, dyskinesia and depression. Other treatable indications are restricted brain function caused by bypass operations or transplants, poor blood supply to the brain, spinal cord injuries, head injuries, hypoxia caused by pregnancy, cardiac arrest and hypoglycaemia. Other treatable indications are protection against liver damage, failure whether drug or disease induced, urinary incontinence, obesity, Fragile-X or Autism.

In addition, it is proposed that mGlu5 receptor antagonists protect against liver damage / failure whether drug or disease induced.

Storto, Marianna; Battaglia, Giuseppe; Gradini, Roberto; Bruno, Valeria; Nicoletti, Ferdinando; Vairetti, Mariapia. Mouse hepatocytes lacking mGlu5 metabotropic glutamate receptors are less sensitive to hypoxic damage. European Journal of Pharmacology (2004), 497(1), 25-27.

Storto, Marianna; Ngomba, Richard Teke; Battaglia, Giuseppe; Freitas, Isabel; Griffini, Patrizia; Richelmi, Plinio; Nicoletti, Ferdinando; Vairetti, Mariapia. Selective blockade of mGlu5 metabotropic glutamate receptors is protective against acetaminophen hepatotoxicity in mice. Journal of Hepatology (2003), 38(2), 179-187.

Storto, Marianna; De Grazia, Ugo; Knopfel, Thomas; Canonico, Pier Luigi; Copani, Agata; Richelmi, Plinio; Nicoletti, Ferdinando; Vairetti, Mariapia. Selective blockade of mGlu5 metabotropic glutamate receptors protects rat hepatocytes against hypoxic damage. Hepatology (Philadelphia) (2000), 31(3), 649-655.

The compounds of formula I, Ia and Ib and their pharmaceutically acceptable salts are especially useful as analgesics. Treatable kinds of pain include inflammatory pain such as arthritis and rheumatoid disease, vasculitis, neuropathic pain such as trigeminal or herpetic neuralgia, diabetic neuropathy pain, causalgia, hyperalgesia, severe chronic pain, post-operative pain and pain associated with various conditions like cancer, angina, renal or billiay colic, menstruation, migraine and gout.

The pharmacological activity of the compounds was tested using the following method:

For binding experiments, cDNA encoding human mGlu 5a receptor was transiently transfected into EBNA cells using a procedure described by Schlaeger and Christensen [Cytotechnology 15:1-13 (1998)]. Cell membrane homogenates were stored at -80°C until the day of assay where upon they were thawed and resuspended and polytronised in 15 mM Tris-HCl, 120 mM NaCl, 100 mM KCl, 25 mM $CaCl_2$, 25 mM $MgCl_2$ binding buffer at pH 7.4 to a final assay concentration of 20 μg protein/ well.

Saturation isotherms were determined by addition of twelve [$^3$H]MPEP concentrations (0.04-100 nM) to these membranes (in a total volume of 200 μl) for 1 h at 4°C. Competition experiments were performed with a fixed concentration of [$^3$H]MPEP (2nM) and $IC_{50}$ values of test compounds evaluated using 11 concentrations (0.3-10,000nM). Incubations were performed for 1 h at 4° C.

At the end of the incubation, membranes were filtered onto unifilter (96-well white microplate with bonded GF/C filter preincubated 1 h in 0.1% PEI in wash buffer, Packard BioScience, Meriden, CT) with a Filtermate 96 harvester (Packard BioScience) and washed 3 times with cold 50 mM Tris-HCl, pH 7.4 buffer. Nonspecific binding was measured in the presence of 10 μM MPEP. The radioactivity on the filter was counted (3 min) on a Packard Top-count microplate scintillation counter with quenching correction after addition of 45 μl of microscint 40 (Canberra Packard S.A., Zürich, Switzerland) and shaking for 20 min.

For functional assays, [$Ca^{2+}$]i measurements were performed as described previously by Porter et al. [Br. J. Pharmacol. 128:13-20 (1999)] on recombinant human mGlu 5a receptors in HEK-293 cells. The cells were dye loaded using Fluo 4-AM (obtainable by FLUKA, 0.2μM final concentration). [$Ca^{2+}$]i measurements were performed using a fluorometric imaging plate reader (FLIPR, Molecular Devices Corporation, La Jolla, CA, USA). Antagonist evaluation was performed following a 5 min preincubation with the test compounds followed by the addition of a sub maximal addition of agonist.

The inhibition (antagonists) curves were fitted with a four parameter logistic equation giving $IC_{50}$, and Hill coefficient using iterative non linear curve fitting software (Xcel fit).

For binding experiments the Ki values of the compounds tested are given. The Ki value is defined by the following formula:

$$K_i = IC_{50} / [1 + L / K_d]$$

in which the $IC_{50}$ values are those concentrations of the compounds tested which cause 50 % inhibition of the competing radioligand ([$^3$H]MPEP). L is the concentration of radioligand used in the binding experiment and the $K_d$ value of the radioligand is empirically determined for each batch of membranes prepared.

The compounds of the present invention are mGluR 5a receptor antagonists. The activities of compounds of formulae I, Ia and Ib as measured in the assay described above and as presented in the table hereafter are in the range of $K_i$ < 250 nM.

| Example | Ki (nM) |
|---|---|
| 1 | 39 |
| 2 | 40 |
| 13 | 14 |
| 26 | 6 |
| 27 | 42 |
| 28 | 211 |

The compounds of formulae I, Ia and Ib and pharmaceutically acceptable salts thereof can be used as medicaments, e.g. in the form of pharmaceutical preparations. The pharmaceutical preparations can be administered orally, e.g. in the form of tablets, coated tablets, dragées, hard and soft gelatine capsules, solutions, emulsions or suspensions. However, the administration can also be effected rectally, e.g. in the form of suppositories, or parenterally, e.g. in the form.of injection solutions.

The compounds of formula I, Ia and Ib and pharmaceutically acceptable salts thereof can be processed with pharmaceutically inert, inorganic or organic carriers for the production of pharmaceutical preparations. Lactose, corn starch or derivatives thereof, talc, stearic acid or its salts and the like can be used, for example, as such as carriers for tablets, coated tablets, dragées and hard gelatine capsules. Suitable carriers for soft gelatine capsules are, for example, vegetable oils, waxes, fats, semi-solid and liquid polyols and the like; depending on the nature of the active substance no carriers are, however, usually required in the case of soft gelatine capsules. Suitable carriers for the production of solutions and syrups are, for example, water, polyols, sucrose, invert sugar, glucose and the like. Adjuvants, such as alcohols, polyols, glycerol, vegetable oils and the like, can be used for aqueous injection solutions of water-soluble salts of compounds of formula I, but as a rule are not necessary. Suitable carriers for suppositories are, for example, natural or hardened oils, waxes, fats, semi-liquid or liquid polyols and the like.

In addition, the pharmaceutical preparations can contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

As mentioned earlier, medicaments containing a compound of formula I or pharmaceutically acceptable salts thereof and a therapeutically inert excipient are also an object of the present invention, as is a process for the production of such medicaments which comprises bringing one or more compounds of formula I or pharmaceutically acceptable salts thereof and, if desired, one or more other therapeutically valuable substances into a galenical dosage form together with one or more therapeutically inert carriers.

The dosage can vary within wide limits and will, of course, be fitted to the individual requirements in each particular case. In general, the effective dosage for oral or parenteral administration is between 0.01-20 mg/kg/day, with a dosage of 0.1-10 mg/kg/day being preferred for all of the indications described. The daily dosage for an adult human being weighing 70 kg accordingly lies between 0.7-1400 mg per day, preferably between 7 and 700 mg per day.

The following examples are provided to further elucidate the invention:

**Example 1**

**2-Chloro-4-[1-(4-fluoro-phenyl)-2,5-dimethyl-1H-imidazol-4-ylethynyl]-pyridine (prepared according to general procedure 3)**

The title compound can be prepared according to general procedure 1 or general procedure 3. The preparation of the title compound according to general procedure 3 is described hereafter in example 1 and the preparation of the title compound according to general procedure 1 is described in example 3.

This compound was prepared according to the general procedure 3 described hereinabove.

2-chloro-4-[1-(4-fluoro-phenyl)-2-methyl-1H-imidazol-4-ylethynyl]-pyridine (200 mg, 0.607 mmol) was dissolved in 10 mL THF and cooled to -75°C. Lithiumdiisopropylamide (0.45 ml, 0.91 mmol) was added and the mixture stirred for 15 min at -75°C. Iodomethane (0.05 ml, 0.85 mmol) was added and stirring was continued at -75 °C for 2 hrs. The reaction mixture was quenched with sat. $NaHCO_3^-$ solution and extracted with water and ethyl acetate. The combined organic extracts were dried with sodium sulfate, filtered and evaporated. The crude product was purified by flash chromatography on silica gel (heptane / ethylacetate 90:10 -> 20:80 gradient) and by recrystallization from ethyl acetate. The desired compound was obtained as a white solid (40 mg, 19%), MS: m/e = 326.5 ($M+H^+$).

**Example 2**

**2-Chloro-4- [1-(2,4-difluoro-phenyl)-2,5-dimethyl-1H-imidazol-4-ylethynyl]-pyridine**

This compound was prepared according to the general procedure 1 described hereinabove.

Step 1: 1-(2,4-Difluoro-phenyl)-2,5-dimethyl-1H-imidazole-4-carboxylic acid ethyl ester

(Z)-2-Acetylamino-3-dimethylamino-but-2-enoic acid ethyl ester (5.5 g, 26 mmol) (Example A) and 2,4-Difluoroaniline (3.3 g, 26 mmol) were stirred at room temperature in acetic acid (45 ml) for 2 hrs. The reaction mixture was evaporated under vacuum at 40°C to obtain 10.3 g dark brown solid [(Z)-2-Acetylamino-3-(2,4-difluoro-phenylamino)-but-2-enoic acid ethyl ester] [MS: m/e = 299.2 (M+H+)], which was refluxed over night at 145°C with fine powdered ammonium sulfate (0.17 g, 1 mmol) in hexamethyldisilazane (64 ml, 306 mmol). The reaction mixture was slowly cooled to 0-5°C. The precipitated solid was filtered and washed with n-hexane to obtain the desired compound as a light brown crystalline solid (1.76 g, 25%), MS: m/e = 281.1 (M+H+).

Step 2: [1-(2,4-Dinuoro-phenyl)-2,5-dimethyl-1H-imidazol-4-yl]-methanol

1-(2,4-Difluoro-phenyl)-2,5-dimethyl-1H-imidazole-4-carboxylic acid ethyl ester (0.5 g, 2 mmol) was dissolved in 15 mL dry THF and cooled to 0°C. Lithium aluminum hydride (2.0 mL, 1M in THF, 2 mmol) was added dropwise and stirred for 45 min at 0°C. The reaction mixture was quenched with 76µl water, 76µl 15% sodium hydroxide and 230µl water. Sodium sulfate was added, stirred for 10 min, filtered and evaporated to dryness to obtain the desired compound as a white solid (0.39 g, 92%), MS: m/e = 239.2 (M+H+).

Step 3: 1-(2,4-Difluoro-phenyl)-2,5-dimethyl-1H-imidazole-4-carbaldehyde

[1-(2,4-Difluoro-phenyl)-2,5-dimethyl-1H-imidazol-4-yl]-methanol (0.38 g, 1.6 mmol) was dissolved in 35 ml dichloromethane. Mangan(IV)oxid (1.38 g,16 mmol) was added and the reaction mixture stirred at reflux for 2 hrs. The suspension was filtered through a dicalite speed plus pad and washed with dichloromethane. The solvents were evaporated and the desired compound was obtained as a yellow oil (0.325 g, 86%), MS: m/e = 237.1 (M+H+).

Step 4: 1-(2,4-Difluoro-phenyl)-4-ethynyl-2,5-dimethyl-1H-imidazole

(1-Diazo-2-oxo-propyl)-phosphonic acid dimethyl ester (0.37 g, 2 mmol) was dissolved in 20 mL methanol. Potassium carbonate (0.38 g, 3 mmol) was added. A solution of 1-(2,4-Difluoro-phenyl)-2,5-dimethyl-1H-imidazole-4-carbaldehyde (0.32 g, 1 mmol) in 5 ml methanol was added drop wise at room temperature. The reaction mixture was stirred at room temperature overnight. The solvent was evaporated. The residue was taken up in 15 ml water and extracted three times with ethyl acetate (15 ml each). The combined organic extracts were dried with sodium sulfate, filtered and evaporated. The crude product was purified by flash chromatography on silica gel (heptane / ethyl acetate 90:10 → 50:50 gradient) and the desired compound was obtained as a light yellow solid (0.165 g, 52%), MS: m/e = 233.1 (M+).

Step 5:2-Chloro-4-[1-(2,4-difluoro-phenyl)-2,5-dimethyl-1H-imidazol-4-ylethynyl]-pyridine

2-Chloro-4-iodo-pyridine (0.21 g, 1 mmol) was dissolved in 10 mL of dry THF and 0.29 mL triethyl amine. This mixture was evacuated and backfilled with argon several times to remove oxygen from the solution. Triphenylphosphine (5 mg, 0.03 eq) and bis(triphenylphosphine)palladium(II)chloride (24 mg, 0.05 eq) were added and the reaction mixture was stirred at room temperature for 10 min. 1-(2,4-Difluoro-phenyl)-4-ethynyl-2,5-dimethyl-1H-imidazole (0.16 g, 1 mmol) and Copper(I)iodide (3 mg, 0.02 eq) were added. The reaction mixture was stirred at room temperature overnight. The solvent was evaporated. The residue was taken up in 10 mL water and extracted three times with ethyl acetate (10 mL each). The combined organic extracts were dried with magnesium sulfate, filtered and evaporated. The crude product was purified by chromatography on silica gel (n-heptane/ethyl acetate 1:1) and recrystallized from diethyl ether. The desired product was obtained as a white solid (0.1 g, 42%), MS: m/e = 344.0 (M+).

**Example 3**

**2-Chloro-4-[1-(4-fluoro-phenyl)-2,5-dimethyl-1H-imidazol-4-ylethynyl]-pyridine (prepared according to general procedure 1)**

The title compound, MS: m/e = 326.2 (M+H+), was prepared in accordance with the general method of example 2 (procedure 1) from (Z)-2-Acetylamino-3-dimethylamino-but-2-enoic acid ethyl ester and 4-Fluoroaniline.

**Example 4**

**2-Chloro-4-[1-(3,5-difluoro-phenyl)-2,5-methyl-1H-imidazol-4-ylethynyl]-pyridine**

The title compound, MS: m/e = 344.0 (M+H+), was prepared in accordance with the general method of example 2 (procedure 1) from (Z)-2-Acetylamino-3-dimethylamino-but-2-enoic acid ethyl ester and 3,5-Difluoroaniline.

**Example 5**

**2-Chloro-4- [1-(4-fluoro-2-methyl-phenyl)-2,5-dimethyl-1H-imidazol-4-ylethynyl]-pyridine**

The title compound, MS: m/e = 340.0 (M+H+), was prepared in accordance with the general method of example 2 (procedure 1) from (Z)-2-Acetylamino-3-dimethylamino-but-2-enoic acid ethyl ester and 4-Fluoro-2-methylaniline.

**Example 6**

**2-Chloro-4-[1-(4-fluoro-3-methyl-phenyl)-2,5-dimethyl-1H-imidazol-4-ylethynyl]-pyridine**

The title compound, MS: m/e = 340.0 (M+H+), was prepared in accordance with the general method of example 2 (procedure 1) from (Z)-2-Acetylamino-3-dimethylamino-but-2-enoic acid ethyl ester and 4-Fluoro-3-methylaniline.

**Example 7**

**2-Chloro-4-(2,5-dimethyl-1-p-tolyl-1H-imidazol-4-ylethynyl)-pyridine**

This compound was prepared according to the general procedure 3 as described hereinabove. 2-Chloro-4-(2-methyl-1-p-tolyl-1H-imidazol-4-ylethynyl)-pyridine (220 mg, 0.71 mmol) was dissolved in 5 mL THF and cooled to -75°C. Lithiumdiisopropylamide (0.61 ml, 1.22 mmol) was added and the mixture stirred for 15 min at -75°C. Iodomethane (0.08 ml, 1.14 mmol) was added and stirring was continued at -75 °C for 2 hrs. The reaction mixture was quenched with sat. $NaHCO_3$ Solution and extracted with water and ethyl acetate. The combined organic extracts were dried with sodium sulfate, filtered and evaporated. The crude product was purified by flash chromatography on silica gel (heptane / ethylacetate 90:10 -> 0:100 gradient) and by recrystallization from dichloromethane and diisopropylether. The desired compound was obtained as a light yellow solid (30 mg, 13%), MS: m/e = 322.3 ($M+H^+$).

**Example 8**

**2-Chloro-4-[1-(3-chloro-4-methyl-phenyl)-2,5-dimethyl-1H-imidazol-4-ylethynyl]-pyridine**

The title compound, MS: m/e = 356.1 (M+H+), was prepared in accordance with the general method of example 2 (procedure 1) from (Z)-2-Acetylamino-3-dimethylamino-but-2-enoic acid ethyl ester and 3-Chloro-4-methylaniline.

**Example 9**

**2-Chloro-4-[1-(3-fluoro-4-methoxy-phenyl)-2,5-dimethyl-1H-imidazol-4-ylethynyl]-pyridine**

The title compound, MS: m/e = 356.1 (M+H+), was prepared in accordance with the general method of example 2 (procedure 1) from (Z)-2-Acetylamino-3-dimethylamino-but-2-enoic acid ethyl ester and 2-Fluoro-p-anisidine.

**Example 10**

**2-Chloro-4-[1-(4-methoxy-phenyl)-2,5-dimethyl-1H-imidazol-4-ylethynyl]-pyridine**

The title compound, MS: m/e = 338.1 (M+H+), was prepared in accordance with the general method of example 2 (procedure 1) from (Z)-2-Acetylamino-3-dimethylamino-but-2-enoic acid ethyl ester and p-Anisidine.

**Example 11**

**2-Chloro-4-[2,5-dimethyl-1-(4-trifluoromethoxy-phenyl)-1H-imidazol-4-ylethynyl]-pyridine.**

The title compound, MS: m/e = 392.1 (M+H+), was prepared in accordance with the general method of example 2 (procedure 1) from (Z)-2-Acetylamino-3-dimethylamino-but-2-enoic acid ethyl ester and 4-(Trifluoromethoxy)aniline.

**Example 12**

**2-Chloro-4-[2,5-dimethyl-1-(3-trifluoromethoxy-phenyl)-1H-imidazol-4-ylethynyl]-pyridine**

The title compound, MS: m/e = 392.1 (M+H+), was prepared in accordance with the general method of example 2 (procedure 1) from (Z)-2-Acetylamino-3-dimethylamino-but-2-enoic acid ethyl ester and 3-(Trifluoromethoxy)aniline.

**Example 13**

**2-Chloro-4-[2,5-dimethyl-1-(4-triffuoromethyl-phenyl)-1H-imidazol-4-ylethynyl]-pyridine**

This compound was prepared according to the general procedure 2 as described hereinabove.

Step 1: 2-Methyl-1-(4-trifluoromethyl-phenyl)-1H-imidazole

2-Methylimidazole (1.0 g, 12 mmol) was dissolved in 35 mL THF. 4-(Trifluoromethyl)phenylboronic acid (2.66 g, 14 mmol) and $[Cu(OH)TMEDA]_2Cl_2$ (1.13 g, 2 mmol) were added. Oxygen was bubbled through the reaction mixture for 30 minutes and stirring was continued at room temperature overnight. The reaction mixture was filtered through a dicalite speed plus pad, washed with 80 ml THF and evaporated. The crude product was purified by flash chromatography on silica gel with ethyl acetate. The desired compound was obtained as a light yellow solid (1.6 g, 58%), MS: m/e = 227.2 (M+H+).

Step 2:4,5-Diiodo-2-methyl-1-(4-trifluoromethyl-phenyl)-1H-imidazole

A mixture of 2-Methyl-1-(4-trifluoromethyl-phenyl)-1H-imidazole (1.6 g, 7.1 mmol), Iodine (2.15 g, 8.5 mmol), Iodic acid (0.75 g, 4.2 mmol), 25 ml acetic acid, 2.5 ml 30% sulfuric acid in water and 4 ml carbon tetrachloride was stirred over night at 80°C. The reaction mixture was cooled to room temperature, decolorized with 5% $NaHSO_3^-$Solution and basified to pH 9 with sodium hydroxide. Water was added and extracted two times with 50 ml ethyl acetate. The organic layers were washed with Brine, dried with sodium sulfate and evaporated. The crude product was recrystalized with little ethyl acetate and cyclohexane. The desired product was obtained as a light yellow solid (1.05 g, 31%). MS:

m/e = 479.0 (M+).

Step 3: 4-Iodo-2,5-dimethyl-1-(4-trinuoromethyl-phenyl)-1H-imidazole

4,5-Diiodo-2-methyl-1-(4-trifluoromethyl-phenyl)-1H-imidazole (1.0 g, 2.1 mmol) was dissolved in 15 ml THF and cooled to -75°C. n-Butyllithium (1.6M in Hexane) (1.60 ml, 2.5 mmol) was added and the mixture stirred for 60 min at -75°C. Iodomethane (0.19 ml, 3.0 mmol) was added and stirring was continued 30 min. at -75 °C and then 1h without dry ice bath. The reaction mixture was quenched with sat. $NaHCO_3^-$ solution and extracted with water and ethyl acetate. The combined organic extracts were dried with sodium sulfate, filtered and evaporated. The crude product was purified by flash chromatography on silica gel (heptane / ethylacetate 100:0 -> 60:40 gradient) and by recrystallization from little ethyl acetate and cyclohexane. The desired compound was obtained as a white solid (197 mg, 26%), MS: m/e = 367.0 ($M+H^+$).

Step 4: 2-Chloro-4-[2,5-dimethyl-1-(4-trifluoromethyl-phenyl)-1H-imidazol-4-ylethynyl]-pyridine

Solution 1: 2-Chloro-4-trimethylsilanylethynyl-pyridine (144 mg, 0.69mmol) (*Example* B) and 4-Iodo-2,5-dimethyl-1-(4-trifluoromethyl-phenyl)-1H-imidazole (180 mg, 0.49 mmol) were dissolved in 3 ml dry THF. This mixture was evacuated and backfilled with argon several times to remove oxygen from the solution.

Solution 2: Triphenylphosphine (4 mg, < 0.1 mmol), bis(triphenylphosphine)-palladium(II)chloride (21 mg, < 0.1 mmol), copper(I)iodide (3 mg, < 0.1 mmol) and triethyl amine (0.1 ml, 0.71 mmol) were dissolved in 4 ml dry THF. This mixture was also evacuated and backfilled with argon several times to remove oxygen from the solution

Solution 2 was heated to 40°C and solution 1 was added dropwise. The reaction mixture was heated to 60°C and tetrabutylammonium fluoride solution (1M in THF, 0.7 ml, 0.7 mmol) was added drop wise. The reaction was then stirred 2 hrs at 60°C. The residue was taken up in 15 ml water and extracted two times with ethyl acetate (15 ml each). The combined organic extracts were dried with magnesium sulfate, filtered and evaporated. The crude product was purified by chromatography on silica gel (heptane/ethyl acetate 1:1) and recrystallized from little ethyl acetate and caclohexane. The desired product was obtained as a white solid (95 mg, 51%), MS: m/e = 376.3 ($M+H^+$).

**Example 14**

**2-Chloro-4-[2,5-dimethyl-1-(3-methyl-4-trifluoromethoxy-phenyl)-1H-imidazol-4-ylethynyl] -pyridine**

The title compound, MS: m/e = 406.2 (M+H+), was prepared in accordance with the general method of example 13 (procedure 2) from 2-Methylimidazole and 3-Methyl-4-(trifluoromethoxy)phenylboronic acid (prepared from 4-Bromo-2-methyl-1-trifluoromethoxy -benzene).

**Example 15**

**2-[4-(2-Chloro-pyridin-4-ylethynyl)-2,5-dimethyl-1H-imidazol-1-yl]-5-methyl-pyridine**

Step 1: 5-Methyl-2-(2-methyl-imidazol-1-yl)-pyridine

2-Methylimidazole (2.0 g, 24 mmol) and 2-Fluoro-5-methylpyridine (5.41 g, 49 mmol) were dissolved in 40 ml dimethyl formamide. Cesium carbonate (23.8 g, 73 mmol) was added and the reaction mixture was stirred at 100°C overnight. The reaction mixture was poured into 100 mL water and extracted three times with ethyl acetate (150 mL each). The combined organic extracts were dried with sodium sulfate, filtered and evaporated. The desired compound was obtained as a brown oil (4.0 g, 95%), MS: m/e = 174.1 (M+H+).

Step 2: 2-[4-(2-Chloro-pyridin-4-ylethynyl)-2,5-dimethyl-imidazol-1-yl]-5-methylpyridine

The title compound, MS: m/e = 323.3 (M+H+), was prepared in accordance with the general method of example 13, step 2, 3 and 4 (procedure 2) from 5-Methyl-2-(2-methyl-imidazol-1-yl)-pyridine.

**Example 16**

**2-Chloro-5-[4-(2-Chloro-pyridin-4-ylethynyl)-2,5-dimethyl-1H-imidazol-1-yl]-pyridine**

The title compound, MS: m/e = 344.1 (M+H+), was prepared in accordance with the general method of example

2 (procedure 1) from (Z)-2-Acetylamino-3-dimethylamino-but-2-enoic acid ethyl ester and 5-Amino-2-chloropyridine.

**Example 17**

**2-[4-(2-Cbloro-pyridin-4-ylethynyl)-2,5-dimethyl-1H-imidazol-1-yl]-6-methyl-4 trifluoromethyl-pyridine**

The title compound, MS: m/e = 3.91.1 (M+H+), was prepared in accordance with the general method of example 15 from 2-Methylimidazole and 2-Chloro-6-methyl-4-(triffuoromethyl)-pyridine.

**Example 18**

**2-[4-(2-Chloro-pyridin-4-ylethynyl)-2,5-dimethyl-1H-irnidazol-1-yl]-pyrazine**

The title compound, MS: m/e = 310.2 (M+H+), was prepared in accordance with the general method of example 15, step 1 and example 12, step 4 from 5-Iodo-2,4-dimethyl-1H-imidazole (see synthesis of intermediates, example C) and 2-Chloropyrazine.

**Example 19**

**2-Chloro-4-[1-(4-chloro-phenyl)-2,5-dimethyl-1H-imidazol-4-ylethynyl]-pyridine**

The title compound, MS: m/e = 342.1 (M+H+), was prepared in accordance with the general method of example 13 (procedure 2) from 2-Methylimidazole and 4-Chlorophenylboronic acid.

**Example 20**

**2-Chloro-4- [1-(3-chloro-2-fluoro-phenyl)-2,5-dimethyl-1H-imidazol-4-ylethynyl]-pyridine**

The title compound, MS: m/e = 360.1 (M+H+), was prepared in accordance with the general method of example 2 (procedure 1) from (Z)-2-Acetylamino-3-dimethylamino-but-2-enoic acid ethyl ester and 3-Chloro-2-fluoroaniline.

**Example 21**

**2-Chloro-4-[2,5-dimethyl-1-(3-trifluoromethyl-phenyl)-1H-imidazol-4-ylethynyl]-pyridine**

The title compound, MS: m/e = 376.3 (M+H+), was prepared in accordance with the general method of example 13 (procedure 2) from 2-Methylimidazole and 3-(Trifluoromethyl)phenylboronic acid.

**Example 22**

**2-Chloro-4-[1-(3-chloro-4-fluoro-phenyl)-2,5-dimethyl-1H-imidazol-4-ylethynyl]-pyridine**

The title compound, MS: m/e = 360.0 (M+H+), was prepared in accordance with the general method of example 2 (procedure 1) from (Z)-2-Acetylamino-3-dimethylamino-but-2-enoic acid ethyl ester and 3-Chloro-4-fluoroaniline.

**Example 23**

**2-[4-(2-Chloro-pyridin-4-ylethynyl)-2,5-dimethyl-1H-imidazol-1-yl]-6-methyl-pyridine**

The title compound, MS: m/e = 323.3 (M+H+), was prepared in accordance with the general method of example 15 from 2-Methylimidazole and 2-Chloro-6-methyl-pyridine.

**Example 24**

**2- [4-(2-Chloro-pyridin-4-ylethynyl)-2,5-dimethyl-1H-imidazol-1-yl]-6-(trifluoromethyl)-pyridine**

The title compound, MS: m/e = 377.1 (M+H+), was prepared in accordance with the general method of example 15 from 2-Methylimidazole and 2-Chloro-6-trifluoromethylpyridine.

**Example 25**

**2-Chloro-4-[2,5-dimethyl-1-(2-methyl-4-trifluoromethoxy-phenyl)-1H-imidazol-4-ylethynyl]-pyridine**

The title compound, was prepared in accordance with the general method of example 2 (procedure 1) from (Z)-2-Acetylamino-3-dimethylamino-but-2-enoic acid ethyl ester and 2-Methyl-4-(trifluoromethoxy)aniline.

**Example 26**

**2-Chloro-4-[5-(4-fluoro-phenyl)-1,4-dimethyl-1H-pyrazol-3-ylethynyl]-pyridine**

The title compound, was prepared in accordance with the general method of general procedure 4 and scheme 4.

Step 1: 1-(1-Ethoxy-vinyl)-4-fluoro-benzene

A solution of 10.0 g 4-Fluoro-iodobenzene, 21.1 g 1-Ethoxyvinyltributyltin and 2.6 g Tetrakis(triphenylphosphine) Palladium in 13.0 ml of Toluene was refluxed overnight under an argon atmosphere. The dark brown solution was filtered over filter aid and concentrated in vaccuo. The crude dark brown oil (7.49 g) was directly used in the next step.

Step 2: [1,1,1-Trichloro-4-ethoxy-4-(4-fluoro-phenyl)-but-3-en-2-one

A solution of 7.49 g 1-(1-Ethoxy-vinyl)-4-Fluorobenzene in 3.6 mL of Pyridine was added at 0°C to 8.19 g of Trichloroacetyl chloride. The suspension was diluted with15 ml of dry methylene chloride and stirred at room temperature overnight. The resulting dark brown suspension was concentrated in vaccuo, filtered over a silicagel column with a 9:1 mixture of Heptane and Ethyl acetate as eluant. One obtains after concentration 28 g of a reddish brown oil which was repurified by chromatography on silicagel using a 19:1 mixture of Heptane and Ethyl acetate as eluant. The obtained brown oil (10.15 g) was directly used in the next step.

Step 3: 5-(4-Fluoro-phenyl)-1-methyl-1H-pyrazole-3-carboxylic acid ethyl ester

A solution of 10.15 g [1,1,1-Trichloro-4-ethoxy-4-(4-fluoro-phenyl)-but-3-en-2-one in 50 ml of Ethanol was added to dropwise to a solution of 2.1 ml of Methylhydrazine and 4.7 ml of 9N HCl/EtOH in 250 ml of Ethanol. The reaction mixture was stirred at reflux for 2 hrs. The yellow solution was allowed to cool and concentrated in vaccuo. The residue was taken up in 200 ml of Methylene chloride. The organic phase was washed successively with IN HCl solution and water. After drying over Magnesium sulfate and concentration, the crude product (8.56 g) was purified by chromatography over silica gel using a 2:1 mixture of Heptane and Ethyl acetate as eluant. The solvents were evaporated and the desired compound was obtained as a yellow oil (2.79 g, 35%), which solidified on standing, MS: m/e = 249.1 ($[M+1]^+$).

Step 4: 5-(4-Fluoro-phenyl)-4-iodo-1-methyl-1H-pyrazole-3-carboxylic acid ethyl ester

To a well strirred solution of 2.79 g of 5-(4-Fluoro-phenyl)-1-methyl-1H-pyrazole-3-carboxylic acid ethyl ester in 100 ml of Acetonitrile were added 1.71 g of Iodine and 3.70 g of Cerium ammonium nitrate. The mixture was stirred for 3 h at 50°C. The red solution was then allowed to cool and was concentrated in vaccuo. The residue was taken up in 200 ml of Ethyl acetate. The organic phase was successively washed with 5% Sodium bisulfite solution and brine. After drying over Magnesium sulfate and concentration, a beige solid 4.06 g (96%) was obtained which is sufficiently pure and was directly used in the next step. MS: m/e = 375.3 ($[M+1]^+$).

Step 5: 5-(4-Fluoro-phepyl)-1,4-climethyl-1H-pyrazole-3-carboxylic acid ethyl ester

To a -78°C solution of 4.06 g of 5-(4-Fluoro-phenyl)-4-iodo-1-methyl-1H-pyrazole-3-carboxylic acid ethyl ester in 80 mL of dry THF were added dropwise 8.13 ml of a 1.6 M solution of n-Butyllithium in THF maintaining the temperature below -70°C. The solution was stirred for 15 min at -75°C and 1.4 ml of Methyl iodide was added. The reaction mixture was stirred for 30 min at -75°C and allowed to warm up to room temperature. The mixture was worked up with Ethyl acetate and brine. The combined organic extracts were dried with magnesium sulfate, filtered and evaporated. The crude product (3.15 g) was purified by chromatography on silica gel (Heptane/Ethyl acetate 2:1). The desired product was obtained as a yellow oil (1.00 g, 35%), which solidified on standing. MS: m/e = 263.1 ([M+1]+).

Step 6:5-(4-Fluoro-phenyl)-1,4-dimethyl-1H-pyrazole-3-carboxylic acid methoxymethyl-amide

To a at 0°C cooled suspension of 0.54 g of N,O-Dimethylhydroxylamine ethyl ester in 10 mL of dry Methylene chloride were added dropwise 2.8 ml of a 2M solution of Trimethylaluminium in Heptane. The solution was stirred for 30 min at room temperature and then cooled to 0°C. Then a solution of 0.65 g 5-(4-Fluoro-phenyl)-1,4-dimethyl-1H-pyrazole-3-carboxylic acid ethyl ester in 4 ml of dry Methylene chloride was added dropwise at 0°C. The solution was allowed to warm up to room temperature and stirred for 3h. The reaction mixture was cooled to 0°C and 20 ml of water were added dropwise. The pH of the aqueous phase was adjusted to 7-8 with IN Sodium hydroxide solution. The mixture was worked up with Methylene chloride and water. The combined organic extracts were dried with magnesium sulfate, filtered and evaporated. The crude product was purified by chromatography on silica gel (Heptane/Ethyl acetate 7:3). The desired product was obtained as a colorless oil (0.52 g, 68%), MS: m/e = 278.0 ([M+1]+).

Step 7: 2-(2-Chloro-pyridin-4-yl)-1-[5-(4-nuoro-phenyl)-1.4-dimethyl-1H-pyrazol-3-yl]-ethanone

To 10 ml of dry THF cooled to -65°C were added 1.35 ml of a 2M solution of Sodium-bis(trimethylsilyl)amide in THF. Then a solution of 0.32 g 2-Chlor-4-methyl pyridine in 1 ml of dry THF was added dropwise maintaining the temperature at -70°C for 30 min. Then a solution of 0.50 g of 5-(4-Fluoro-phenyl)-1,4-dimethyl-1H-pyrazole-3-carboxylic acid methoxymethyl-amide in 5 ml of dry THF was added dropwise over a 10 min period maintaining the temperature below -65 °C. The solution was stirred for 2h at -60°C, and then allowed to warm up to 0°C and stirred for 20 min at this temperature. Then 0.31 ml of Acetic acid was added dropwise at -50°C and the solution was allowed to warm up to room temperature and stirred overnight. The mixture was concentrated in vaccuo. The residue was taken up in Ethyl acetate. The organic phase was washed with saturated bicarbonate solution and brine, dried over magnesium sulfate, filtered and evaporated. The crude product (0.67 g, yellow oil) was purified by chromatography on silica gel (Heptane/Ethyl acetate 4:1). The desired product was obtained as a white solid (0.27 g, 70%), MS: m/e = 344.0 ([M+1]+).

Step 8:3-Chloro-2-(2-chloro-pyridin-4-yl)-3-[5-(4-fluoro-phenyl)-1,4-dimeth-1H-pyrazol-3-yl]-propenal

To 15 ml of dry Methylene chloride cooled to 0°C was added 0.34 ml of Chloromethylene-dimethylimidium chloride. Then a solution of 0.35 g 2-(2-Chloro-pyridin-4-yl)-1-[5-(4-fluoro-phenyl)-1,4-dhnethyl-1H-pyrazol-3-yl]-ethanone in 6 ml of dry Methylene chloride was added at 0°C over a period of 5 min. The yellow suspension was stirred for 2.5 h at 0°C, and the diluted with 20 ml of Methylene chloride. The organic phase was washed with saturated bicarbonate solution and water, dried over magnesium sulfate, filtered and evaporated. The crude product (0.42 g, light yellow solid) was directly used in the next step, MS: m/e = 389.1 (M+).

Step 9: 2-Chloro-4-[5-(4-fluoro-phenyl)-1,4-dimethyl-1H-pyrazol-3-ylethynyl]-pyridine

To 11 ml of THF cooled to 0°C were added 0.26 g of Potassium tert-butylate and 20 μl of water.

Then a solution of 0.40 g 3-Chloro-2-(2-chloro-pyridin-4-yl)-3-[5-(4-fluoro-phenyl)-1,4-dimethyl-1H-pyrazol-3-yl]-propenal in 7 ml of THF was added at 0°. After stirring for 1 h at 5°C, 10 ml of 5% Bicarbonate solution were added and the aqueous phase was extracted twice with Ethyl acetate. The combined organic phases were washed with water and brine, dried over magnesium sulfate, filtered and evaporated. The crude product (0.28 g, yellow oil) was purified by chromatography on silica gel (Heptane/Ethyl acetate 4:1). The desired product was obtained as a white solid (0.275 g, 82%), MS: m/e = 326.0 (M+).

**Example 27**

**2-Chloro-4-[5-difluoromethyl-1-(4-fluoro-phenyl)-2-methyl-1H-imidazol-4-ylethynyl]-pyridine**

This compound was prepared according to the general procedure 3 described hereinabove.

Step1: 5-(2-Chloro-pyridin-4-ylethynyl)-3-(4-fluoro-phenyl)-2-methyl-3H-imidazole-4-carbaldehyde:

2-chloro-4-[1-(4-fluoro-phenyl)-2-methyl-1H-imidazol-4-ylethynyl]-pyridine (2.0 g, 6.42 mmol) was dissolved in 50 mL THF and cooled to -70°C. Lithiumdiisopropylamide 2M/THF (4.8 ml, 9.6 mmol) was added and the mixture stirred for 15 min at -70°C. Dimethylformamide (0.69 ml, 9.0 mmol) was added and stirring was continued at -70 °C for 3 hrs. The reaction mixture was quenched with sat. $NaHCO_3^-$ solution and extracted with water and ethyl acetate. The combined organic extracts were dried with sodium sulfate, filtered and evaporated. The crude product was purified by flash chromatography on silica gel (heptane / ethylacetate 1:2). The desired compound was obtained as a yellow solid (220 mg,

10%), MS: m/e = 340.0 ($M+H^+$).

Step2: 2-Chloro-4-[5-difluoromethyl-1-(4-fluoro-phenyl)-2-methyl-1H-imidazol-4-ylethynyl]-pyridine:

To a solution of 100 mg (0.29 mmol) 5-(2-Chloro-pyridin-4-ylethynyl)-3-(4-fluorophenyl)-2-methyl-3H-imidazole-4-carbaldehyde in 3 ml of dry methylene chloride were added 125 mg (0.775 mmol) Diethylaminosulfur trifluoride (DAST). The mixture was stirred at room temperature for 3d. The reaction mixture was quenched with sat. $NaHCO_3$- solution and extracted with water and methylene chloride. The combined organic extracts were dried with sodium sulfate, filtered and evaporated. The crude product was purified by flash chromatography on silica gel (heptane / ethyl acetate 1:1). The desired compound was obtained as a light yellow solid (47 mg, 44%), MS: m/e = 362.1 ($M+H^+$).

**Example 28**

**[5-(2-Chloro-pyridin-4-ylethynyl)-3-(4-fluoro-phenyl)-2-methyl-3H-imidazol-4-yl]-methanol**

To a solution of 100 mg (0.29 mmol) 5-(2-Chloro-pyridin-4-ylethynyl)-3-(4-fluorophenyl)-2-methyl-3H-imidazole-4-carbaldehyde (example 26) in 5 ml of methanol were added at 0°C 11 mg (0.29 mmol) of sodium borohydride, and the mixture was stirred for 1h at 0°C. The reaction mixture was quenched with sat. $NaHCO_3^-$ solution, and evaporated. The residue was extracted with water and ethyl acetate. The combined organic extracts were dried with sodium sulfate, filtered and evaporated. The crude product was purified by flash chromatography on silica gel (heptane / ethyl acetate 1:1). The desired compound was obtained as a white solid (40 mg, 40%), MS: m/e = 342.1 ($M+H^+$).

**Example 29**

**2-Chloro-4-[1-(4-methoxy-3-trifluoromethyl-phenyl)-2,5-dimethyl-1H-imidazol-4-ylethynyl] -pyridine**

This compound was prepared according to the general procedure 5 described hereinabove.

Step 1: 1-(4-Methoxy-3-trifluoromethyl-phenyl)-2,5-dimethyl-1H-imidazole-4-carboxylic acid ethyl ester

Ethyl 2-Hydroxyimino-3-oxobutanoate (1.67 g, 10.5 mmol) (Example C), 3-trifluoromethyl-4-methoxyaniline (2.0 g, 10.5 mmol) and pyridinium p-toluenesulfonate (0.13 g, 0.52 mmol) were stirred at 75°C in toluene (15 ml) for 4 hrs. The reaction mixture was evaporated under vacuum at 40°C. The residue was dissolved in triethyl orthoacetate (14.3 ml, 78 mmol) and p-toluenesulfonic acid monohydrate (0.1 g, 0.52 mmol) and palladium on charcoal (0.6 g) were added. The reaction mixture was stirred for 4 hrs under hydrogen atmosphere. The dark suspension was filtered and evaporated to dryness. The crude product was recrystallized at 0°C from TBME and n-heptane. The desired product was obtained as a light brown solid (1.28 g, 36%), MS: m/e = 343.1 (M+H+).

Step 2:2-(2-Chloro-pyridin-4-yl)-1-[1-(4-methoxy-3-trifluoromethyl-phenyl)-2,5-dimethyl-1H-imidazol-4-yl]-ethanone.

1-(4-Methoxy-3-trifluoromethyl-phenyl)-2,5-dimethyl-1H-imidazole-4-carboxylic acid ethyl ester (0.8 g, 2.3 mmol) and 2-chloro-4-methylpyridine (0.36 g, 2.8 mmol) were dissolved in 10 mL toluene. This solution was dropped to a cold (0°C) mixture of potassium bis(trimethylsilyl)amide (1.17 g, 5.84 mmol) in 15 mL toluene. The reaction mixture was sirred for 90 min. at 0°C. 0.4 mL acetic acid were added and the mixture extracted with water, saturated $NaHCO_3$-Solution and brine. The aqueous layer were washed with toluene (100 mL). The combined organic ex-tracts were dried with sodium sulfate and filtered. The solvent was evaporated and the crude product [(0.8 g, 81%), [MS: m/e = 424.5 (M+H+)] was used without any further purification for the next step.

Step 3: 3-Chloro-2-(2-chloro-pyridin-4-yl)-3-[1-(4-methoxy-3-trifluoromethyl-phenyl)-2,5-dimeihyl-1H-imidazol-4-yl]-propenal

A solution of 2-(2-Chloro-pyridin-4-yl)-1-[1-(4-methoxly-3-trifluoromethyl-phenyl)-2,5-dimethyl-1H-imidazol-4-yl]-ethanone (0.8 g,1.9 mmol) in 8 mL dichloromethane was dropped to a cold (0°C) suspension of chloromethylenedimethyliminium chloride (0.6 g, 4.7 mmol) in 4 mL dichloromethane. The reaction mixture was stirred at 0°C for 1 hr. The reaction was diluted with 10 mL water and basified with saturated $NaHCO_3$-Solution to pH 8. The layers were separated and the aqueous layer was extracted with 100 mL dichloromethane. The organic layers were washed with water, combined and dried over magnesium sulfate. The solvent was evaporated and the crude product [(0.9 g, 100%), MS: m/e = 470.3 (M+H+)] was used without any further purification for the next step.

Step 4: 2-Chloro-4-[1-(4-meihoxy-3-trifluoromethyl-phenyl)-2,5-dimethyl-1H-imidazol-4-ylethynyl]pyridine

A solution of 3-Chloro-2-(2-chloro-pyridin-4-yl)-3-[1-(4-methoxy-3-trifluoromethylphenyl)-2,5-dimethyl-1H-imidazol-4-yl]-propenal (0.9 g, 1.9 mmol) in 8 mL THF was dropped to a cold (0°C) suspension of potassium tert-butoxide (0.47 g, 4.2 mmol) in 4 mL THF and water (0.04 mL, 2.1 mmol). The light brown reaction mixture was stirred at 0°C for 1 hr. The reaction was extracted two times with ethyl acetate, saturated $NaHCO_3$-Solution, water and brine. The combined organic extracts were dried with sodium sulfate, filtered and evaporated. The crude product was purified by chromatography on silica gel (n-heptane/ethyl acetate 90:10 -> 0:100) to obtained the desired product as a white solid (0.15 g, 19%), MS: m/e = 406.2 (M+H+).

**Example 30**

**2-Chloro-4-[1-(3,5-diuuoro-4-methoxy-phenyl)-2,5-dimethyl-1H-imidazol-4-ylethynyl]-pyridine**

The title compound [MS: m/e = 373.9 (M+H+)], was prepared in accordance with the general method of example 29 (general procedure 5) from Ethyl 2-Hydroxyimino-3-oxobutanoate (Example C) and 3,5-Difluoro-4-methoxyaniline (Example D).

**Example 31**

**2-Chloro-4-[1-(4-methoxy-3-tritluoromethoxy-phenyl)-2,5-dimethyl-1H-imidazol-4-ylethynyl]-pyridine**

The title compound [MS: m/e = 421.9 (M+H+)], was prepared in accordance with the general method of example 29 (general procedure 5) from Ethyl 2-Hydroxyimino-3-oxobutanoate (Example C) and 4-Methoxy-3-trifluoromethoxyaniline (Example E).

**Example 32**

**2-Chloro-4-[1-(3-methoxy-4-trifluoromethoxly-phenyl)-2,5-dinlethyl-1H-inlidazol-4-ylethynyl] -pyridine**

The title compound [MS: m/e = 421.9 (M+H+)], was prepared in accordance with the general method of example 29 (general procedure 5) from Ethyl 2-Hydroxyimino-3-oxobutanoate (Example C) and 3-Methoxy-4-trifluoromethoxyaniline (Example F).

**Example 33**

**3-[4-(2-Chloro-pyridin-4-ylethynyl)-2,5-dimethyl-1H-imidazol-1-yl]-5-fluoro-pyridine**

The title compound, MS: m/e = 327.0 (M+H+), was prepared in accordance with the general method of example 15 from 2-Methylimidazole and 3,5-diffuoropyridine.

**Example 34**

**4-{3-[4-(2-Chloro-pyridin-4-ylethynyl)-2,5-dimethyl-imidazol-1-yl]-5-fluoro-phenyl}-morpholine**

A mixture of 2-Chloro-4-[1-(3,5-difluoro-phenyl)-2,5-methyl-1H-imidazol-4-ylethynyl]-pyridine (0.08 g, 0.23 mmol) (Example 4), morpholine (0.041 g, 0.46 mmol) and potassium carbonate (0.13 g, 0.92 mmol) in 1 mL DMSO was stirred at 100°C for 60 hrs. The reaction mixture was cooled and extracted two times with ethyl acetate and water. The organic layers were washed with brine, combined and dried over sodium sulfate. The solvent was evaporated and the crude product was purified by chromatography on silica gel (n-heptane/ethyl acetate 90:10 -> 30:70) and recrystallized from diisopropylether to obtained the desired product as a white solid (8.0 mg, 8%), MS: m/e = 411.2 (M+H+).

**Example 35**

**2-Chloro-4-[1-(4-fluoro-2-trifluoromethoxy-phenyl)-2,5-dimethyl-1H-imidazol-4-ylethynyl]-pyridine**

The title compound, MS: m/e = 410.0 (M+H+), was prepared in accordance with the general method of example 2 (procedure 1) from (Z)-2-Acetylamino-3-dimethylamino-but-2-enoic acid ethyl ester and 4-Fluoro-2-trifluoromethoxyaniline (Example G).

**Example 36**

**2-Chloro-4-[1-(2-fluoro-4-trifluoromethoxy-phenyl)-2,5-dimethyl-1H-imidazol-4-ylethynyl]-pyridine**

The title compound, MS: m/e = 410.1 (M+H+), was prepared in accordance with the general method of example 2 (procedure 1) from (Z)-2-Acetylamino-3-dimethylamino-but-2-enoic acid ethyl ester and 2-Fluoro-4-trifluoromethoxyaniline (Example H).

**Exampe 37**

The following examples can also be prepared as according to general procedure 1 or general procedure 5:

2-Chloro-4-[2,5-dimethyl-1-(4-methyl-3-trifluoromethyl-phenyl)-1H-imidazol-4-ylethynyl] -pyridine;
2-Chloro-4- [2,5-dimethyl-1-(3-methyl-4-trifluoromethyl-phenyl)-1H-imidazol-4-ylethynyl] -pyridine;
2-Chloro-4-[2,5-dimethyl-1-(3-methyl-5-trifluoromethyl-phenyl)-1H-imidazol-4-ylethynyl] -pyridine;
2-Chloro-4- [1-(3-methoxy-5-trifluoromethyl-phenyl)-2,5-dimethyl-1H-imidazol-4-ylethynyl] -pyridine;
2-Chloro-4-[1-(3-methoxy-4-trifluoromethyl-phenyl)-2,5-dimethyl-1H-imidazol-4-ylethynyl] -pyridine;
2-Chloro-4- [1-(3,5-dichloro-phenyl)-2,5-dimethyl-1H-imidazol-4-ylethynyl]-pyridine;
2-Chloro-4- [1-(3-chloro-5-methyl-phenyl)-2,5-dimethyl-1H-imidazol-4-ylethynyl]-pyridine;
2-Chloro-4- [1-(3-fluoro-5-methyl-phenyl)-2,5-dimethyl-1H-imidazol-4-ylethynyl]-pyridine;
2-Chloro-4-[1-(3-chloro-5-methoxy-phenyl)-2,5-dimethyl-1H-imidazol-4-ylethynyl]-pyridine; and
2-Chloro-4-[1-(3-fluoro-5-methoxy-phenyl)-2,5-dimethyl-1H-imidazol-4-ylethynyl]-pyridine.

**Synthesis of Intermediates**

**Example A**

**(Z)-2-Acetylamino-3-dimethylamino-but-2-enoic acid ethyl ester**

In this example of the compound X, $R^2$ and $R^4$ are both methyl. Nevertheless, it is understood that the person skilled in the art would be able to prepare other compounds of formula X, wherein $R^2$ and $R^4$ are other than methyl using the method of the following example:

Step 1: 4-[1-Dimethylamino-eth-(Z)-ylidene]-2-methyl-4H-oxazol-5-one

N-Acetylglycine (10.0 g, 85.4 mmol) and Phosphoroxychloride (19.6 ml, 213.5 mmol) were mixed and cooled to 5°C. N',N-Dimethylacetamide (19.7 ml, 213.5 mmol) was added drop-wise slowly during 30 min at 5-10°C (exothermic!). The reaction mixture was stirred at 45°C for 2 hrs and then cooled to room temperature. Dichloromethane (35 ml) was added and the mixture poured into 200 ml ice-water. The pH was adjusted to pH 8 with ammonium hydroxide and the mixture was extracted twice with 50 ml dichloromethane. The organic extracts were washed with 30 ml water, dried over magnesium sulfate, filtered and evaporated. The crude product was purified by column chromatography on silica gel (ethyl acetate) and the desired compound was obtained as a light brown solid (7.40 g, 51%), MS: m/e = 169.2 (M+H+).

Step 2: (Z)-2-Acetylamino-3-dimethylamino-but-2-enoic acid ethyl ester

4-[1-Dimethylamino-eth-(Z)-ylidene]-2-methyl-4H-oxazol-5-one (7.4 g, 44.0 mmol) was dissolved in ethanol (50 ml) and sodium hydride (0.10g, 4.4 mmol) was added at room temperature. The dark solution was refluxed for 1h. The solvent was evaporated and the crude product [MS: m/e = 215.5 (M+H+)] was used without any further purification for the next step.

**Example B**

**5-Iodo-2,4-dimethyl-1H-imidazole**

In this example of the compound XIX, $R^2$ is methyl, $R^4$ is methyl, and X is chloro. Nevertheless it is understood that the person skilled in the art would be able to prepare other compounds of formula XIX, wherein $R^2$ and $R^4$ are other

than methyl, and X is other than chloro, using the method used for in the following example: 2,4-Dimethylimidazole (5.0g, 52 mmol) was suspended in 100 ml Acetonitrile and N-Iodosuccinimide (14.0g, 62.4 mmol) was added. The reaction mixture was stirred at reflux for 16 hours, then evaporated and decolorized with sat $NaHSO_3^-$ solution. Water was added and extracted two times with 100 ml ethyl acetate. The organic layers were washed with Brine, dried with sodium sulfate and evaporated. The crude product was purified by chromatography on silica gel (dichloromethane/methanol 9:1). The desired product was obtained as a light brown solid (5.10 g, 44%), MS: m/e = 223.0 ($M+H^+$).

**Example C**

**Ethyl 2-Hydroxyimino-3-oxobutanoate**

The title compound was prepared from ethyl acetoacetate in accordance with the literature reference of Robinson, Stanislawski & Mulholland, The Journal of Organic Chemistry, Volume 66, Number 12, 4148-4152 (2001).

**Example D**

**3,5-Difluoro-4-methoxyaniline**

The title compound was prepared from 2,6-difluorophenol in accordance with the literature reference of Qiu, Stevenson, O'Beirne and Silverman, J. Med. Chem. 1999, 42, 329-332.

**Example E**

**4-Methoxy-3-trifluoromethoxyaniline**

The title compound can be prepared in accordance with patent WO 2004007444.

**Example F**

**3-Methoxy-4-trifluoromethoxyaniline**

The title compound can be prepared in accordance with patent WO 9613492.

**Example G**

**4-Fluoro-2-trifluoromethoxyaniline**

The title compound can be prepared in accordance with patent EP 318704.

**Example H**

**2-Fluoro-4-trifluoromethoxyaniline**

The title compound can be prepared in accordance with patent EP 318704.

Preparation of the pharmaceutical compositions:

Example I

Tablets of the following composition are produced in a conventional manner:

| | mg/Tablet |
|---|---|
| Active ingredient | 100 |
| Powdered. lactose | 95 |
| White corn starch | 35 |
| Polyvinylpyrrolidone | 8 |
| Na carboxymethylstarch | 10 |

(continued)

| | |
|---|---|
| Magnesium stearate | 2 |
| Tablet weight | 250 |

Example II

Tablets of the following composition are produced in a conventional manner:

| | mg/Tablet |
|---|---|
| Active ingredient | 200 |
| Powdered. lactose | 100 |
| White corn starch | 64 |
| Polyvinylpyrrolidone | 12 |
| Na carboxymethylstarch | 20 |
| Magnesium stearate | 4 |
| Tablet weight | 400 |

Example III

Capsules of the following composition are produced:

| | mg/Capsule |
|---|---|
| Active ingredient | 50 |
| Crystalline, lactose | 60 |
| Microcrystalline cellulose | 34 |
| Talc | 5 |
| Magnesium stearate | 1 |
| Capsule fill weight | 150 |

The active ingredient having a suitable particle size, the crystalline lactose and the microcrystalline cellulose are homogeneously mixed with one another, sieved and thereafter talc and magnesium stearate are admixed. The final mixture is filled into hard gelatine capsules of suitable size.

## Claims

1. A compound of the general formula

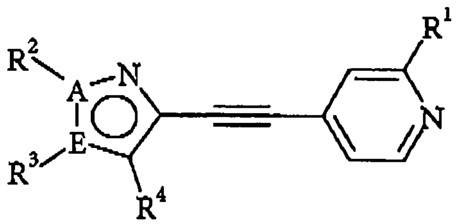

(I)

wherein
one of A or E is N and the other is C;

$R^1$ is halogen or cyano;
$R^2$ is $C_1$-$C_6$ alkyl;
$R^3$ is phenyl or heteroaryl, which are optionally substituted by:

one, two or three substituents, selected from the group consisting of halogen, $C_1$-$C_6$ alkyl, $C_1$-$C_6$ alkoxy, cycloalkyl, $C_1$-$C_6$ haloalkyl, $C_1$-$C_6$ haloalkoxy, cyano, NR'R" or by 1-morpholinyl, or by
1-pyrrolidinyl, optionally substituted by $(CH_2)_{0,1}OR$, or by
piperidinyl, optionally substituted by $(CH_2)_{0,1}OR$, or by
1,1-dioxo-thiomorpholinyl or by
piperazinyl, optionally substituted by $C_1$-$C_6$ alkyl or $(CH_2)_{0,1}$-cycloalkyl,

wherein the heteroaryl is an aromatic 5- or 6-membered ring containing one or more heteroatoms selected from nitrogen, oxygen, or sulfur;
R is hydrogen, $C_1$-$C_6$alkyl or $(CH_2)_{0,1}$-cycloalkyl;
R', R" are independently from each other hydrogen, $C_1$-$C_6$ alkyl, $(CH_2)_{0,1}$-cycloallcyl or $(CH_2)_nOR$;
n is 1 or 2;
$R^4$ is $CHF_2$, $CF_3$, or C(O)H, $CH_2R^5$ wherein $R^5$ is hydrogen, OH, $C_1$-$C_6$-alkyl or $C_3$-$C_{12}$-cycloalkyl;

as well as to pharmaceutically acceptable salts thereof.

**2.** The compound of claim 1, wherein the compound is of formula Ia:

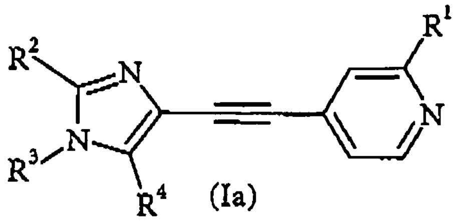

(Ia)

wherein $R^1$, $R^2$, $R^3$ and $R^4$ are as defined in claim 1 as well as to pharmaceutically acceptable salts thereof.

**3.** The compound of claim 1, wherein the compound is of formula Ib:

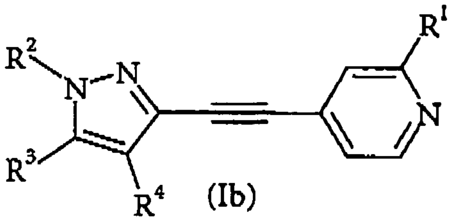

(Ib)

wherein $R^1$, $R^2$, $R^3$ and $R^4$ are as defined in claim 1 as well as to pharmaceutically acceptable salts thereof.

**4.** A compound according to any one of claim 2 or 3, wherein

$R^1$ is halogen;
$R^2$ is methyl or i-propyl;
$R^3$ is selected from phenyl, pyridinyl, pyrazinyl, pyrimidinyl or pyridazinyl which may be substituted by one or more chloro, fluoro, $C_1$-$C_6$ alkyl, $C_1$-$C_6$ alkoxy, cyano, $C_1$-$C_6$ haloalkyl, $C_1$-$C_6$haloalkoxy or cycloalkyl; and
$R^4$ is $CHF_2$; $CH_2R^5$ wherein $R^5$ is hydrogen, OH or $C_1$-$C_6$-alkyl;

as well as pharmaceutically acceptable salts thereof.

**5.** A compound of formula Ia in accordance with claim 2, wherein $R^3$ is unsubstituted or substituted heteroaryl, and wherein the substituents are selected from chloro, fluoro, $CF_3$, and $C_1$-$C_6$ alkyl.

**6.** A compound of formula Ia in accordance with claim 5 which is selected from the group consisting of:

2-[4-(2-Chloro-pyridin-4-ylethynyl)-2,5-dimethyl-1H-imidazol-1-yl]-5-methyl-pyridine;

2-Chloro-5-[4-(2-Chloro-pyridin-4-ylethynyl)-2,5-dimethyl-1H-innidazol-1-yl]-pyridine;
2-[4-(2-Chloro-pyridin-4-ylethynyl)-2,5-dimethyl-1H-imidazol-1-yl]-6-methyl-4-trifluoromethyl-pyridine;
2-[4-(2-Chloro-pyridin-4-ylethynyl)-2,5-dimethyl-1H-imidazol-1-yl]-pyrazine;
2-[4-(2-Chloro-pyricdin-4-ylethynyl)-2,5-dimethyl-1H-imidazol-1-yl]-6-methyl-pyridine;
2-[4-(2-Chloro-pyridin-4-ylethynyl)-2,5-dimethyl-1H-imidazol-1-yl]-6-(trifluoromethyl)-pyridine; and
3-[4-(2-Chloro-pyridin-4-ylethynyl)-2,5-dimethyl-1H-imidazol-1-yl]-5-fluoro-pyridine.

**7.** A compound of formula Ia in accordance with claim 2, wherein $R^3$ is aryl, substituted by one or more chloro, fluoro, $CF_3$, $C_1$-$C_6$ alkyl, $C_1$-$C_6$ alkoxy, $CF_3O$ or 1-morpholinyl.

**8.** A compound of formula Ia in accordance with claim 7 which is selected from the group consisting of:

2-Chloro-4-[1-(4-fluoro-phenyl)-2,5-dimethyl-1H-imidazol-4-ylethynyl]-pyridine;
2-Chloro-4-[1-(2,4-difluoro-phenyl)-2,5-dimethyl-1H-imidazol-4-ylethynyl]-pyridine;
2-Chloro-4-[1-(3,5-difluoro-phenyl)-2,5-dirnethyl-1H-imidazol-4-ylethynyl]-pyridine;
2-Chloro-4-[1-(4-fluoro-2-meoyl-phenyl)-2,5-dimeoyl-1H-imidazol-4-ylethynyl]-pyridine;
2-Chloro-4-[1-(4-fluoro-3-methyl-phenyl)-2,5-dimethyl-1H-imidazol-4-ylethynyl]-pyridine;
2-Chloro-4-(2,5-dimethyl-1-p-tolyl-1H-imidazol-4-ylethynyl)-pyridine;
2-Chloro-4-[1-(3-chloro-4-methyl-phenyl)-2,5-dimethyl-1H-imidazol-4-ylethynyl] -pyridine;
2-Chloro-4-[1-(3-fluoro-4-methoxy-phenyl)-2,5-dimethyl-1H-imidazol-4-ylethynyl] -pyridine;
2-Chloro-4-[1- (4-methoxy-phenyl)-2,5-dimethyl-1H-imidazol-4-ylethynyl]-pyridine;
2-Chloro-4-[2,5-dimethyl-1-(4-trinuoromethoxy-phenyl)-1 H-imidazol-4-ylethynyl] -pyridine;
2-Chloro-4-[2,5-dimethyl-1-(3-triffuoromethoxy-phenyl)-1H-imidazol-4-ylethynyl]-pyridine;
2-Chloro-4-[2,5-dimethyl-1-(4-trifluoromeoyl-phenyl)-1H-imidazol-4-ylethynyl]-pyridine;
2-Chloro-4-[2,5-dirnethyl-1-(3-methyl-4-trifluoromethoxy-phenyl)-1H-imxdazol-4-ylethynyl]-pyridine;
2-Chloro-4-[1-(4-clxloro-phenyl)-2,5-dimethyl-1H-imidazol-4-ylethynyl]-pyridine;
2-Chloro-4-[1-(3-chloro-2-fluoro-phenyl)-2,5-dimethyl-1H-irnidazol-4-ylethynyl]-pyridine;
2-Chloro-4-[2,5-dimethyl-1-(3-triuuoromethyl-phenyl)-1H-imidazol-4-ylethynyl]-pyridine;
2-Chloro-4-[1-(3-chloro-4-nuoro-phenyl)-2,5-dimethyl-1H-imidazol-4-ylethynyl]-pyridine;
2-Chloro-4-[2,5-dimethyl-1-(2-methyl-4-trifluoromethoxy-phenyl)-1H-infidazol-4-ylethynyl]-pyridine;
2-Chloro-4-[5-diauoromethyl-1-(4-fluoro-phenyl)-2-methyl-1H-imidazol-4-ylethynyl]-pyridine;
[5-(2-Chloro-pyridin-4-ylethynyl)-3-(4-fluoro-phenyl)-2-methyl-3H-imidazol-4-yl]-methanol;
2-Chloro-4-[1-(4-methoxy-3-trifluoromethyl-phenyl)-2,5-dimethyl-1H-imidazol-4-ylethynyl]-pyridine;
2-Chloro-4-[1-(3,5-difluoro-4-methoxy-phenyl)-2,5-dimethyl-1H-imidazol-4-ylethynyl]-pyridine;
2-Chloro-4-[1-(4-methoxy-3-trifluoromethoxy-phenyl)-2,5-dimethyl-1H-imidazol-4-ylethynyl]-pyridine;
2-Chloro-4-[1-(3-methoxy-4-trifluoromethoxy-phenyl)-2,5-dimethyl-1H-imidazol-4-ylethynyl]-pyridine;
4-{3-[4-(2-Chloro-pyridin-4-ylethynyl)-2,5-dimethyl-imidazol-1-yl]-5-fluoro-phenyl}-morpholine;
2-Chloro-4-[1-(4-fluoro-2-trifiuoromethoxy-phenyl)-2,5-dimethyl-1H-imidazol-4-ylethynyl]-pyridine;
2-Chloro-4-[1-(2-fluoro-4-trifluofomethoxy-phenyl)-2,5-dimethyl-1H -îmidazol-4- ylethynyl]-pyridine;
2-Chloro-4-[2,5-dimethyl-1-(4-methyl-3-trifluoromethyl-phenyl)-1H-imidazol-4-ylethynyl]-pyridine;
2-Chloro-4-[2,5-dimethyl-1-(3-methyl-4-trinuoromethyl-phenyl)-1H-imidazol-4-ylethynyl]-pyridine;
2-Chloro-4-[2,5-dimethyl-1-(3-methyl-5-trifluoromethyl-phenyl)-1H-imidazol-4-ylethynyl]-pyridine;
2-Chloro-4-[1-(3-methoxy-5-trifluoromethyl-phenyl)-2,5-dimethyl-1H-imidazol-4-ylethynyl]-pyridine;
2-Chloro-4-[1-(3-methoxy-4-trifluoromethyl-phenyl)-2,5-dimethyl-1H-imidazol-4-ylethynyl]-pyridine;
2-Chloro-4-[1-(3,5-dichloro-phenyl)-2,5-dimethyl-1H-imidazol-4-ylethynyl]-pyridine;
2-Chloro-4-[1-(3-chloro-5-methyl-phenyl)-2,5-dimethyl-1H-imidazol-4-ylethynyl]-pyridine;
2-Chloro-4-[1-(3-fluoro-5-methyl-phenyl)-2,5-dimethyl-1H-imidazol-4-ylethynyl] -pyridine;
2-Chloro-4-[1-(3-chloro-5-methoxy-phenyl)-2,5-dimethyl-1H-imidazol-4-ylethynyl] -pyridine; and
2-Chloro-4- [1-(3-fluoro-5-methoxy-phenyl)-2,5-dimethyl-1H-imidazol-4-ylethynyl]-pyridine.

**9.** A compound of formula Ib in accordance with claim 3, wherein wherein $R^3$ is aryl, substituted by one or more fluoro.

**10.** A compound of formula Ib in accordance with claim 9, which is 2-Chloro-4-[5-(4-fluoro-phenyl)-1,4-dimethyl-1H-pyrazol-3-ylethynyl] -pyridine,

**11.** A process for preparing a compound of formula la according to claim 2, which process comprises reacting a compound of formula II

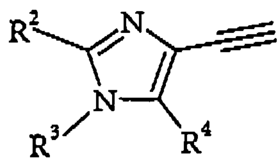

(II)

with a compound of formula III

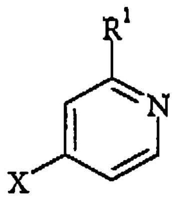

(III)

in order to obtain the compound of formula Ia;
wherein $R^1$, $R^2$, $R^3$ and $R^4$ are as defined in claim 1 and X is halogen, and if desired, converting the compounds obtained into pharmaceutically acceptable acid addition salts.

**12.** A process for preparing a compound of formula Ia as defined in claim 2, which process comprises reacting a compound of formula IV

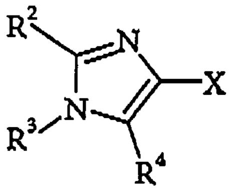

(IV)

with a compound of formula V

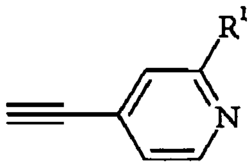

(V)

in order to obtain the compound of formula Ia;
wherein $R^1$, $R^2$, $R^5$ and $R^4$ are as defined in claim 1 and X is halogen, and if desired, converting the compounds obtained into pharmaceutically acceptable acid addition salts.

**13.** A process for preparing a compound of formula Ia as defined in claim 2, which process comprises reacting a compound of formula Ic

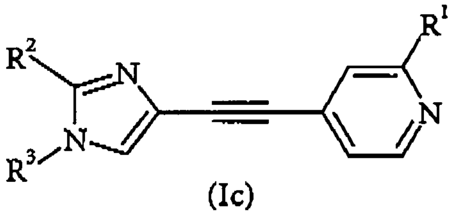

(Ic)

with a compound of formula VI

$R^4$-X (VI)

in order to obtain the compound of formula Ia;
wherein $R^1$, $R^2$, $R^3$ and $R^4$ are as defined in claim 1 and X is halogen, and if desired, converting the compounds obtained into pharmaceutically acceptable acid addition salts.

**14.** A process for preparing a compound of formula Ib as defined in claim 3, which process comprises reacting a compound of formula XXVI

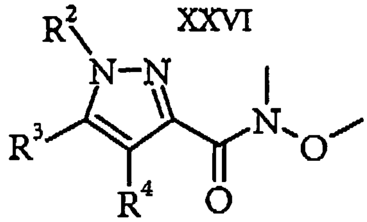
XXVI

with a compound of formula XXVII

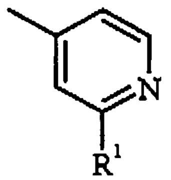
XXVII

in order to obtain a compound of formula XXVIII

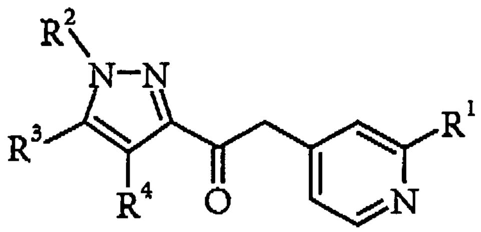
XXVIII

and convert the compound of formula XXVIII into the compound of formula Ib;
wherein $R^1$, $R^2$, $R^3$ and $R^4$ are as defined in claim 1, and if desired, converting the compounds obtained into pharmaceutically acceptable acid addition salts.

**15.** A medicament containing one or more compounds as claimed in any one of claims 1 to 10 and pharmaceutically acceptable excipients for the treatment and prevention of mGluR5 receptor mediated disorders.

**16.** A medicament according to claim 15 for the treatment and prevention of acute and/or chronic neurological disorders, in particular anxiety, or for the treatment of chronic and acute pain, protection against liver damage or failure whether drug or disease induced, urinary incontinence, obesity, Fragile-X or Autism.

**17.** A compound in accordance with any one of claims 1 to 10 as well as its pharmaceutically acceptable salt for use in the treatment or prevention of diseases.

**18.** The use of a compound in accordance with any one of claims 1 to 10 as well as its pharmaceutically acceptable salt for the manufacture of medicaments for the treatment and prevention of acute and chronic neurological disorders,

cognitive disorders, memory deficits, acute and chronic pain, epilepsy, schizophrenia, anxiety, acute, traumatic or chronic degenerative processes of the nervous system, Alzheimer's disease, senile dementia, Huntington's chorea, amyotrophic lateral sclerosis, multiple sclerosis, dementia caused by AIDS, eye injuries, retinopathy, idiopathic parkinsonism, parkinsonism caused by medicaments, muscle spasms, convulsions, migraine, urinary incontinence, ethanol addiction, nicotine addiction, psychoses, opiate addiction, anxiety, vomiting, dyskinesia, depression, restricted brain function caused by bypass operations, poor blood supply to the brain, spinal cord injuries, head injuries, hypoxia caused by pregnancy, cardiac arrest and hypoglycemia, liver damage, failure whether drug or disease induced, obesity, Fragile-X or Autism, and urinary incontinence.

## Patentansprüche

1. Eine Verbindung der allgemeinen Formel

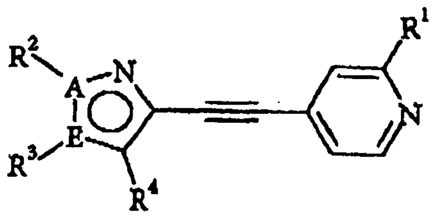

(I)

wobei
einer von A oder E Stickstoff und der andere Kohlenstoff ist;

$R^1$ Halogen oder Cyano ist;
$R^2$ $C_1$-$C_6$-Alkyl ist;
$R^3$ Phenyl oder Heteroaryl ist, das gegebenenfalls substituiert ist mit:

einem, zwei oder drei Substituenten, ausgewählt aus der Gruppe bestehend aus Halogen, $C_1$-$C_6$-Alkyl, $C_1$-$C_6$-Alkoxy, Cycloalkyl, $C_1$-$C_6$-Halogenalkyl, $C_1$-$C_6$-Halogenalkoxy, Cyano, NR'R'' oder mit
1-Morpholinyl oder mit
1-Pyrrolidinyl, gegebenenfalls substituiert mit $(CH_2)_{0,1}$OR oder mit
Piperidinyl, gegebenenfalls substituiert mit $(CH_2)_{0,1}$OR oder mit
1,1-Dioxothiomorpholinyl oder mit
Piperazinyl, gegebenenfalls substituiert mit $C_1$-$C_6$-Alkyl oder $(CH_2)_{0,1}$-Cycloalkyl,

wobei das Heteroaryl ein aromatischer 5- oder 6-gliedriger Ring ist, der ein oder mehrere Heteroatome enthält, ausgewählt aus Stickstoff, Sauerstoff oder Schwefel;
R Wasserstoff, $C_1$-$C_6$-Alkyl oder $(CH_2)_{0,1}$-Cycloalkyl ist;
R', R'' unabhängig voneinander Wasserstoff, $C_1$-$C_6$-Alkyl, $(CH_2)_{0,1}$-Cycloalkyl oder $(CH_2)_n$OR sind;
n 1 oder 2 ist;
$R^4$ $CHF_2$, $CF_3$ oder C(O)H, $CH_2R^5$ ist, wobei $R^5$ Wasserstoff, OH, $C_1$-$C_6$-Alkyl oder $C_3$-$C_{12}$-Cycloalkyl ist;

sowie auch pharmazeutisch verträgliche Salze davon.

2. Verbindung nach Anspruch 1, wobei die Verbindung die Formel Ia aufweist:

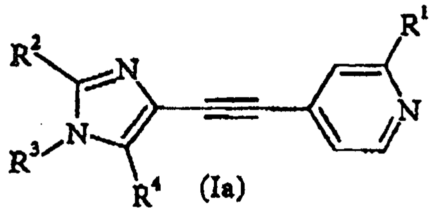

(Ia)

wobei $R^1$, $R^2$, $R^3$ und $R^4$ wie in Anspruch 1 definiert sind, sowie auch pharmazeutisch verträgliche Salze davon.

**3.** Verbindung nach Anspruch 1, wobei die Verbindung die Formel Ib aufweist:

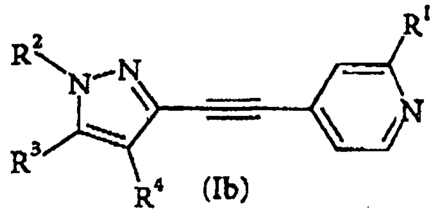

(Ib)

wobei $R^1$, $R^2$, $R^3$ und $R^4$ wie in Anspruch 1 definiert sind, sowie auch pharmazeutisch verträgliche Salze davon.

**4.** Verbindung gemäß einem der Ansprüche 2 oder 3, wobei

$R^1$ Halogen ist;
$R^2$ Methyl oder i-Propyl ist;
$R^3$ ausgewählt ist aus Phenyl, Pyridinyl, Pyrazinyl, Pyrimidinyl oder Pyridazinyl, die mit einem oder mehreren von Chlor, Fluor, $C_1$-$C_6$-Alkyl, $C_1$-$C_6$-Alkoxy, Cyano, $C_1$-$C_6$-Halogenalkyl, $C_1$-$C_6$-Halogenalkoxy oder Cycloalkyl substituiert sein können; und
$R^4$ $CHF_2$ oder $CHR^5$ ist, wobei $R^5$ Wasserstoff, OH oder $C_1$-$C_6$-Alkyl ist;

sowie auch pharmazeutisch verträgliche Salze davon.

**5.** Verbindung der Formel Ia gemäß Anspruch 2, wobei $R^3$ unsubstituiertes oder substituiertes Heteroaryl ist und wobei die Substituenten ausgewählt sind aus Chlor, Fluor, $CF_3$ und $C_1$-$C_6$-Alkyl.

**6.** Verbindung der Formel Ia gemäß Anspruch 5, die ausgewählt ist aus der Gruppe bestehend aus:

2-[4-(2-Chlorpyridin-4-ylethinyl)-2,5-dimethyl-1H-imidazol-1-yl]-5-methylpyridin;
2-Chlor-5-[4-(2-Chlorpyridin-4-ylethinyl)-2,5-dimethyl-1H-imidazol-1-yl]pyridin;
2-[4-(2-Chlorpyridin-4-ylethinyl)-2,5-dimethyl-1H-imidazol-1-yl]-6-methyl-4-trifluormethylpyridin;
2-[4-(2-Chlorpyridin-4-ylethinyl)-2,5-dimethyl-1H-imidazol-1-yl]pyrazin;
2-[4-(2-Chlorpyridin-4-ylethinyl)-2,5-dimethyl-1H-imidazol-1-yl]-6-methylpyridin;
2-[4-(2-Chlorpyridin-4-ylethinyl)-2,5-dimethyl-1H-imidazol-1-yl]-6-(trifluormethyl)pyridin;
und
3-[4-(2-Chlorpyridin-4-ylethinyl)-2,5-dimethyl-1H-imidazol-1-yl]-5-fluorpyridin;

**7.** Verbindung der Formel Ia gemäß Anspruch 2, wobei $R^3$ Aryl ist, das mit einem oder mehreren Chlor, Fluor, $CF_3$, $C_1$-$C_6$-Alkyl, $C_1$-$C_6$-Alkoxy, $CF_3O$ oder 1-Morpholinyl substituiert ist.

**8.** Verbindung der Formel Ia gemäß Anspruch 5, die ausgewählt ist aus der Gruppe bestehend aus:

2-Chlor-4-[1-(4-fluorphenyl)-2,5-dimethyl-1H-imidazol-4-ylethinyl]pyridin;
2-Chlor-4-[1-(2,4-difluorphenyl)-2,5-dimethyl-1H-imidazol-4-ylethinyl]pyridin;
2-Chlor-4-[1-(3,5-difluorphenyl)-2,5-dimethyl-1H-imidazol-4-ylethinyl]pyridin;

2-Chlor-4-[1-(4-fluor-2-methylphenyl)-2,5-dimethyl-1H-imidazol-4-ylethinyl]pyridin;
2-Chlor-4-[1-(4-fluor-3-methylphenyl)-2,5-dimethyl-1H-imidazol-4-ylethinyl]pyridin;
2-Chlor-4-(2,5-dimethyl-1-p-tolyl-1H-imidazol-4-ylethinyl)pyridin;
2-Chlor-4-[1-(3-chlor-4-methylphenyl)-2,5-dimethyl-1H-imidazol-4-ylethinyl]pyridin;
2-Chlor-4-[1-(3-fluor-4-methoxyphenyl)-2,5-dimethyl-1H-imidazol-4-ylethinyl]pyridin;
2-Chlor-4-[1-(4-methoxyphenyl)-2,5-dimethyl-1H-imidazol-4-ylethinyl]pyridin;
2-Chlor-4-[2,5-dimethyl-1-(4-trifluormethoxyphenyl)-1H-imidazol-4-ylethinyl]pyridin;
2-Chlor-4-[2,5-dimethyl-1-(3-trifluormethoxyphenyl)-1H-imidazol-4-ylethinyl]pyridin;
2-Chlor-4-[2,5-dimethyl-1-(4-trifluormethylphenyl)-1H-imidazol-4-ylethinyl]pyridin;
2-Chlor-4-[2,5-dimethyl-1-(3-methyl-4-trifluormethoxyphenyl)-1H-imidazol-4-ylethinyl]pyridin;
2-Chlor-4-[1-(4-chlorphenyl)-2,5-dimethyl-1H-imidazol-4-ylethinyl]pyridin;
2-Chlor-4-[1-(3-chlor-2-fluorphenyl)-2,5-dimethyl-1H-imidazol-4-ylethinyl]pyridin;
2-Chlor-4-[2,5-dimethyl-1-(3-trifluonnethylphenyl)-1H-imidazol-4-ylethinyl]pyridin;
2-Chlor-4-[1-(3-chlor-4-fluorphenyl)-2,5-dimethyl-1H-imidazol-4-ylethinyl]pyridin;
2-Chlor-4-[2,5-dimethyl-1-(2-methyl-4-trifluormethoxyphenyl)-1H-imidazol-4-ylethinyl]pyridin;
2-Chlor-4-[5-difluormethyl-1-(4-fluorphenyl)-2-methyl-1H-imidazol-4-ylethinyl]pyridin;
[5-(2-Chlorpyridin-4-ylethinyl)-3-(4-fluorphenyl)-2-methyl-3H-imidazol-4-yl]methanol;
2-Chlor-4-[1-(4-methoxy-3-trifluormethylphenyl)-2,5-dimethyl-1H-imidazol-4-ylethinyl]pyridin;
2-Chlor-4-[1-(3,5-difluor-4-methoxyphenyl)-2,5-dimethyl-1H-imidazol-4-ylethinyl]pyridin;
2-Chlor-4-[1-(4-methoxy-3-trifluormethoxyphenyl)-2,5-dimethyl-1H-imidazol-4-ylethinyl]pyridin;
2-Chlor-4-[1-(3-methoxy-4-trifluormethoxyphenyl)-2,5-dimethyl-1H-imidazol-4-ylethinyl]pyridin;
4-{3-[4-(2-Chlorpyridin-4-ylethinyl)-2,5-dimethylimidazol-1-yl]-5-fluorphenyl}morpholin;
2-Chlor-4-[1-(4-fluor-2-trifluormethoxyphenyl)-2,5-dimethyl-1H-imidazol-4-ylethinyl]pyridin;
2-Chlor-4-[1-(2-fluor-4-trifluormethoxyphenyl)-2,5-dimethyl-1H-imidazol-4-ylethinyl]pyridin;
2-Chlor-4-[2,5-dimethyl-1-(4-methyl-3-trifluormethylphenyl)-1H-imidazol-4-ylethinyl]pyridin;
2-Chlor-4-[2,5-dimethyl-1-(3-methyl-4-trifluormethylphenyl)-1H-imidazol-4-ylethinyl]pyridin;
2-Chlor-4-[2,5-dimethyl-1-(3-methyl-5-trifluormethylphenyl)-1H-imidazol-4-ylethinyl]pyridin;
2-Chlor-4-[1-(3-methoxy-5-trifluorrnethylphenyl)-2,5-dimethyl-1H-imidazol-4-ylethinyl]pyridin;
2-Chlor-4-[1-(3-methoxy-4-trifluormethylphenyl)-2,5-dimethyl-1H-imidazol-4-ylethinyl]pyridin;
2-Chlor-4-[1-(3,5-dichlorphenyl)-2,5-dimethyl-1H-imidazol-4-ylethinyl]pyridin;
2-Chlor-4-[1-(3-chlor-5-methylphenyl)-2,5-dimethyl-1H-imidazol-4-ylethinyl]pyridin;
2-Chlor-4-[1-(3-fluor-5-methylphenyl)-2,5-dimethyl-1H-imidazol-4-ylethinyl]pyridin;
2-Chlor-4-[1-(3-chlor-5-methoxyphenyl)-2,5-dimethyl-1H-imidazol-4-ylethinyl]pyridin;
und
2-Chlor-4-[1-(3-fluor-5-methoxyphenyl)-2,5-dimethyl-1H-imidazol-4-ylethinyl]pyridin.

**9.** Verbindung der Formel Ib gemäß Anspruch 3, wobei wobei $R^3$ Aryl, ist, das mit einem oder mehreren Fluor substituiert ist.

**10.** Verbindung der Formel Ib gemäß Anspruch 9, nämlich 2-Chlor-4-[5-(4-fluorphenyl)-1,4-dimethyl-1H-pyrazol-3-ylethinyl]pyridm.

**11.** Verfahren zur Herstellung einer Verbindung der Formel Ia gemäß Anspruch 2, wobei das Verfahren das Umsetzen einer Verbindung der Formel II

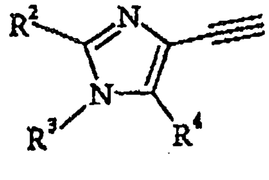

(II)

mit einer Verbindung der Formel III

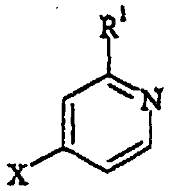

(III)

zu der Verbindung der Formel Ia umfasst;
wobei $R^1$, $R^2$, $R^3$ und $R^4$ wie in Anspruch 1 definiert sind und X Halogen ist und, falls es gewünscht wird, das Überführen der erhaltenen Verbindungen in pharmazeutisch verträgliche Säureadditionssalze.

**12.** Verfahren zur Herstellung einer Verbindung der Formel Ia, wie in Anspruch 2 definiert, wobei das Verfahren das Umsetzen einer Verbindung der Formel IV

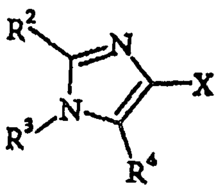

(IV)

mit einer Verbindung der Formel V

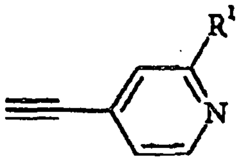

(V)

zu der Verbindung der Formel Ia umfasst;
wobei $R^1$, $R^2$, $R^3$ und $R^4$ wie in Anspruch 1 definiert sind und X Halogen ist und, falls es gewünscht wird, das Überführen der erhaltenen Verbindungen in pharmazeutisch verträgliche Säureadditionssalze.

**13.** Verfahren zur Herstellung einer Verbindung der Formel Ia, wie in Anspruch 2 definiert, wobei das Verfahren das Umsetzen einer Verbindung der Formel Ic

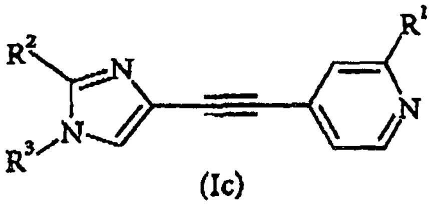

(Ic)

mit einer Verbindung der Formel VI

$R^4$-X (VI)

zu der Verbindung der Formel Ia umfasst;
wobei $R^1$, $R^2$, $R^3$ und $R^4$ wie in Anspruch 1 definiert sind und X Halogen ist und, falls es gewünscht wird, das

Überführen der erhaltenen Verbindungen in pharmazeutisch verträgliche Säureadditionssalze.

**14.** Verfahren zur Herstellung einer Verbindung der Formel Ib, wie in Anspruch 3 definiert, wobei das Verfahren das Umsetzen einer Verbindung der Formel XXVI

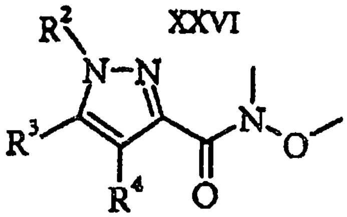
XXVI

mit einer Verbindung der Formel XXVII

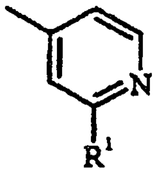
XXVII

zu einer Verbindung der Formel XXVIII;

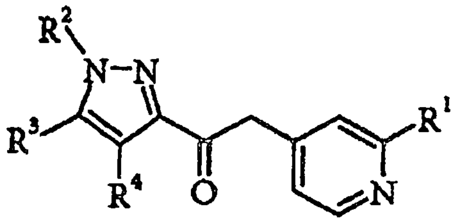
XXVIII

und das Überführen der Verbindung der Formel XXVIII in die Verbindung der Formel Ib umfasst; wobei $R^1$, $R^2$, $R^3$ und $R^4$ wie in Anspruch 1 definiert sind und X Halogen ist und, falls es gewünscht wird, das Überführen der erhaltenen Verbindungen in pharmazeutisch verträgliche Säureadditionssalze.

**15.** Medikament, das eine oder mehrere Verbindungen, wie sie in einem der Ansprüche 1 bis 10 beansprucht werden und pharmazeutisch verträgliche Excipienten enthält, zur Behandlung und Prophylaxe von mGluR5-Rezeptor vermittelten Störungen.

**16.** Medikament gemäß Anspruch 15 zur Behandlung und Prophylaxe von akuten und/oder chronischen neurologischen Störungen, insbesonders von Angstzuständen oder zur Behandlung von chronischen und akuten Schmerzen, zum Schutz gegen Leberschäden oder fehlern, unabhängig davon ob durch Drogen oder Krankheit verursacht, Harninkontinenz, Fettleibigkeit, Fragiles-X-Syndrom oder Autismus.

**17.** Verbindung gemäß einem der Ansprüche 1 bis 10 sowie ihre pharmazeutisch verträglichen Salze zur Verwendung bei der Behandlung oder Prophylaxe von Krankheiten.

**18.** Verwendung einer Verbindung gemäß einem der Ansprüche 1 bis 10 sowie ihrer pharmazeutisch verträglichen Salze zur Herstellung von Medikamenten zur Behandlung und Prophylaxe von akuten und chronischen neurologischen Störungen, kognitiven Störungen, Gedächtnisschwächen, akutem und chronischem Schmerz, Epilepsie, Schizophrenie, Angstzuständen, akuten, traumatischen oder chronischen degenerativen Prozessen des Nervensystems, Alzheimer'scher Krankheit, Altersdemenz, Chorea-Huntington, amyotropher Lateralsklerose, Multiple Sklerose, Demenz, die durch AIDS hervorgerufen wird, Augenverletzungen, Retinopathie, idiopathische Parkinson'sche Krankheit, Parkinson'scher Krankheit, die durch Medikamente hervorgerufen wird, Muskelspasmen, Konvulsionen, Migräne, Harninkontinenz, Alkoholabhängigkeit, Nikotinabhängigkeit, Psychosen, Opiatabhängigkeit, Angstzustände, Erbrechen, Dyskinesie, Depression, eingeschränkte Hirnfunktionen, die durch Bypassoperationen hervorgerufen werden, ungenügende Blutversorgung des Gehirns, Rückenmarksverletzungen, Kopfverletzungen, Hypoxie, die durch Schwangerschaft hervorgerufen wird, Herzstillstand und Hypoglykämie, Leberschäden, -fehler durch Drogen oder durch Krankheit hervorgerufen, Fettleibigkeit, Fragilem-X-Syndrom oder Autismus und Harninkontinenz.

## Revendications

**1.** Composé de la formule générale :

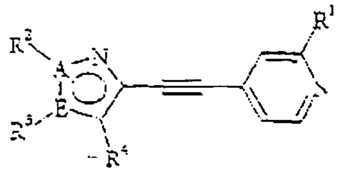

(I)

dans laquelle

l'un de A ou de E est N et l'autre est C ;
$R^1$ est un halogène ou un cyano ;
$R^2$ est un alkyle en $C_1$ à $C_6$ ;
$R^3$ est un phényle ou un hétéroaryle, qui sont éventuellement substitués par :

un, deux ou trois substituants, choisis parmi le groupe constitué par un halogène, un alkyle en $C_1$ à $C_6$, un alcoxy en $C_1$ à $C_6$, un cycloalkyle, un halogénoalkyle en $C_1$ à $C_6$, un halogénoalcoxy en $C_1$ à $C_6$, un cyano, NR'R" ou par :

un 1-morpholinyle, ou par
un 1-pyrrolidinyle, éventuellement substitué par $(CH_2)_{0,1}OR$, ou par
un pipéridinyle, éventuellement substitué par $(CH_2)_{0,1}OR$, ou par
un 1,1-dioxo-thiomorpholinyle ou par
un pipérazinyle, éventuellement substitué par un alkyle en $C_1$ à $C_6$ ou $(CH_2)_{0,1}$-cycloalkyle ;

le groupe hétéroaryle étant un cycle aromatique de 5 à 6 chaînons contenant un ou plusieurs hétéroatomes choisis parmi un atome d'azote, d'oxygène ou de soufre ;

R est un atome d'hydrogène, un groupe alkyle en $C_1$ à $C_6$ ou $(CH_2)_{0,1}$-cycloalkyle ;
R', R" sont indépendamment l'un de l'autre de l'hydrogène, un alkyle en $C_1$ à $C_6$, un $(CH_2)_{0,1}$-cycloalkyle ou un $(CH_2)_nOR$ ;
n est 1 ou 2 ;
$R^4$ est $CHF_2$, $CF_3$ ou C(O)H, $CH_2R^5$ où $R^5$ est de l'hydrogène, OH, un alkyle en $C_1$-$C_6$ ou un cycloalkyle en $C_3$-$C_{12}$ ;

ainsi que leurs sels pharmaceutiquement acceptables.

**2.** Composé selon la revendication 1, dans lequel le composé est de formule Ia :

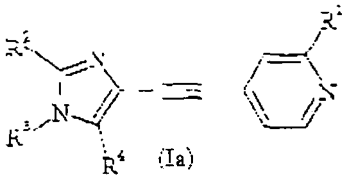

dans laquelle $R^1$, $R^2$, $R^3$ et $R^4$ sont tels que définis dans la revendication 1 ainsi que ses sels pharmaceutiquement acceptables.

**3.** Composé selon la revendication 1, dans lequel le composé est de formule Ib :

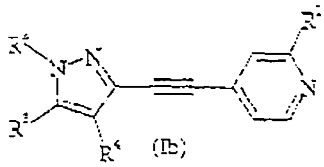

dans laquelle $R^1$, $R^2$, $R^3$ et $R^4$ sont tels que définis dans la revendication 1 ainsi que ses sels pharmaceutiquement acceptables.

**4.** Composé selon l'une quelconque des revendications 2 ou 3, dans lequel

$R^1$ est un halogène ;
$R^2$ est un groupe méthyle ou i-propyle ;
$R^3$ est choisi parmi un groupe phényle, pyridinyle, pyrazinyle, pyrimidinyle ou pyridazinyle qui peut être substitué par un ou plusieurs chloro, fluoro, alkyle en $C_1$ à $C_6$, alcoxy en $C_1$ à $C_6$, cyano, halogénoalkyle en $C_1$ à $C_6$, halogénoalcoxy en $C_1$ à $C_6$ ou cycloalkyle ; et
$R^4$ est $CHF_2$ ; $CH_2R^5$ où $R^5$ est de l' hydrogène, OH ou un alkyle en $C_1$-$C_6$ ;

ainsi que ses sels pharmaceutiquement acceptables.

**5.** Composé de formule Ia selon la revendication 2, dans laquelle $R^3$ est un groupe hétéroaryle non substitué ou substitué, et dans laquelle les substituants sont choisis parmi un chloro, fluoro, $CF_3$, et alkyle en $C_1$ à $C_6$.

**6.** Composé de formule Ia selon la revendication 5 qui est choisi parmi le groupe constitué par :

la 2-[4-(2-chloropyridin-4-yléthynyl)-2,5-diméthyl-1H-imidazol-1-yl]-5-méthylpyridine ;
la 2-chloro-5-[4-(2-chloropyridin-4-yléthynyl)-2,5-diméthyl-1H-imidazol-1-yl]-pyridine ;
la 2-[4-(2-chloropyridin-4-yléthynyl)-2,5-diméthyl-1H-imidazol-1-yl]-6-méthyl-4-trifluorométhyl-pyridine ;
la 2-[4-(2-chloropyridin-4-yléthynyl)-2,5-diméthyl-1H-imidazol-1-yl]-pyrazine ;
la 2-[4-(2-chloropyridin-4-yléthynyl)-2,5-diméthyl-1H-imidazol-1-yl]-6-méthylpyridine ;
la 2-[4-(2-chloropyridin-4-yléthynyl)-2,5-diméthyl-1H-imidazol-1-yl]-6-(trifluorométhyl)-pyridine ; et
la 3-[4-(2-chloropyridin-4-yléthynyl)-2,5-diméthyl-1H-imidazol-1-yl]-5-fluoropyridine.

**7.** Composé de formule Ia selon la revendication 2, dans laquelle $R^3$ est un groupe aryle, substitué par un ou plusieurs chloro, fluoro, $CF_3$, alkyle en $C_1$-$C_6$, alcoxy en $C_1$-$C_6$, $CF_3O$ ou 1-morpholinyle.

**8.** Composé de formule Ia selon la revendication 7 qui est choisi dans le groupe constitué par :

la 2-chloro-4-[1-(4-fluorophényl)-2,5-diméthyl-1H-imidazol-4-yléthynyl]-pyridine ;
la 2-chloro-4-[1-(2,4-difluorophényl)-2,5-diméthyl-1H-imidazol-4-yléthynyl]-pyridine ;
la 2-chloro-4-[1-(3,5-difluorophényl)-2,5-diméthyl-1H-imidazol-4-yléthynyl]-pyridine ;
la 2-chloro-4-[1-(4-fluoro-2-méthylphényl)-2,5-diméthyl-1H-imidazol-4-yléthynyl]-pyridine ;
la 2-chloro-4-[1-(4-fluoro-3-méthylphényl)-2,5-diméthyl-1H-imidazol-4-yléthynyl]-pyridine ;

la 2-chloro-4-(2,5-diméthyl-1-p-tolyl-1H-imidazol-4-yléthynyl)-pyridine ;
la 2-chloro-4-[1-(3-chloro-4-méthylphényl)-2,5-diméthyl-1H-imidazol-4-yléthynyl]-pyridine ;
la 2-chloro-4-[1-(3-fluoro-4-méthoxyphényl)-2,5-diméthyl-1H-imidazol-4-yléthynyl]-pyridine ;
la 2-chloro-4-[1-(4-méthoxyphényl)-2,5-diméthyl-1H-imidazol-4-yléthynyl]-pyridine ;
la 2-chloro-4-[2,5-diméthyl-1-(4-trifluorométhoxyphényl)-1H-imidazol-4-yléthynyl]-pyridine ;
la 2-chloro-4-[2,5-diméthyl-1-(3-trifluorométhoxyphényl)-1H-imidazol-4-yléthynyl]-pyridine ;
la 2-chloro-4-[2,5-diméthyl-1-(4-trifluorométhylphényl)-1H-imidazol-4-yléthynyl]-pyridine ;
la 2-chloro-4-[2,5-diméthyl-1-(3-méthyl-4-trifluorométhoxyphényl)-1H-imidazol-4-yléthynyl]-pyridine ;
la 2-chloro-4-[1-(4-chlorophényl)-2,5-diméthyl-1H-imidazol-4-yléthynyl]-pyridine ;
la 2-chloro-4-[1-(3-chloro-2-fluorophényl)-2,5-diméthyl-1H-imidazol-4-yléthynyl]-pyridine ;
la 2-chloro-4-[2,5-diméthyl-1-(3-trifluorométhylphényl)-1H-imidazol-4-yléthynyl]-pyridine ;
la 2-chloro-4-[1-(3-chloro-4-fluorophényl)-2,5-diméthyl-1H-imidazol-4-yléthynyl]-pyridine ;
la 2-chloro-4-[2,5-diméthyl-1-(2-méthyl-4-trifluorométhoxyphényl)-1H-imidazol-4-yléthynyl]-pyridine ;
la 2-chloro-4-[5-difluorométhyl-1-(4-fluorophényl)-2-méthyl-1H-imidazol-4-yléthynyl]-pyridine ;
le [5-(2-chloropyridin-4-yléthynyl)-3-(4-fluorophényl)-2-méthyl-3H-imidazol-4-yl]-méthanol ;
la 2-chloro-4-[1-(4-méthoxy-3-trifluorométhylphényl)-2,5-diméthyl-1H-imidazol-4-yléthynyl]-pyridine ;
la 2-chloro-4-[1-(3,5-difluoro-4-méthoxyphényl)-2,5-diméthyl-1H-imidazol-4-yléthynyl]-pyridine ;
la 2-chloro-4-[1-(4-méthoxy-3-trifluorométhoxyphényl)-2,5-diméthyl-1H-imidazol-4-yléthynyl]-pyridine ;
la 2-chloro-4-[1-(3-méthoxy-4-trifluorométhoxyphényl)-2,5-diméthyl-1H-imidazol-4-yléthynyl]-pyridine ;
la 4-{3-[4-(2-chloropyridin-4-yléthynyl)-2,5-diméthyl-imidazol-1-yl]-5-fluorophényl}morpholine ;
la 2-chloro-4-[1-(4-fluoro-2-trifluorométhoxyphényl)-2,5-diméthyl-1H-imidazol-4-yléthynyl]-pyridine ;
la 2-chloro-4-[1-(2-fluoro-4-trifluorométhoxyphényl)-2,5-diméthyl-1H-imidazol-4-yléthynyl]-pyridine ;
la 2-chloro-4-[2,5-diméthyl-1-(4-méthyl-3-trifluorométhylphényl)-1H-imidazol-4-yléthynyl]-pyridine ;
la 2-chloro-4-[2,5-diméthyl-1-(3-méthyl-4-trifluorométhylphényl)-1H-imidazol-4-yléthynyl]-pyridine ;
la 2-chloro-4-[2,5-diméthyl-1-(3-méthyl-5-trifluorométhylphényl)-1H-imidazol-4-yléthynyl]-pyridine ;
la 2-chloro-4-[1-(3-méthoxy-5-trifluorométhylphényl)-2,5-diméthyl-1H-imidazol-4-yléthynyl]-pyridine ;
la 2-chloro-4-[1-(3-méthoxy-4-trifluorométhylphényl)-2,5-diméthyl-1H-imidazol-4-yléthynyl]-pyridine ;
la 2-chloro-4-[1-(3,5-dichlorophényl)-2,5-diméthyl-1H-imidazol-4-yléthynyl]-pyridine ;
la 2-chloro-4-[1-(3-chloro-5-méthylphényl)-2,5-diméthyl-1H-imidazol-4-yléthynyl]-pyridine ;
la 2-chloro-4-[1-(3-fluoro-5-méthylphényl)-2,5-diméthyl-1H-imidazol-4-yléthynyl]-pyridine ;
la 2-chloro-4-[1-(3-chloro-5-méthoxyphényl)-2,5-diméthyl-1H-imidazol-4-yléthynyl]-pyridine ; et
la 2-chloro-4-[1-(3-fluoro-5-méthoxyphényl)-2,5-diméthyl-1H-imidazol-4-yléthynyl]-pyridine.

**9.** Composé de formule Ib selon la revendication 3, dans laquelle $R^3$, est un groupe aryle, substitué par un ou plusieurs fluoro.

**10.** Composé de formule Ib selon la revendication 9, qui est la 2-chloro-4-[5-(4-fluorophényl)-1, 4-diméthyl-1H-pyrazol-3-yléthynyl]-pyridine.

**11.** Procédé de préparation d'un composé de formule Ia selon la revendication 2, lequel procédé comprend la réaction d'un composé de formule II

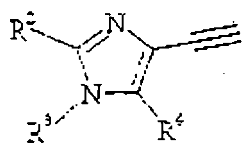 (II)

avec un composé de formule III

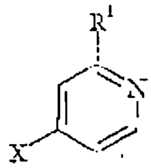 (II)

afin d'obtenir un composé de formule Ia ; où $R^1$, $R^2$, $R^3$ et $R^4$ sont tels que définis dans la revendication 1 et X est un halogène, et si souhaité, la conversion des composés obtenus en sels d'addition à un acide pharmaceutiquement acceptables.

**12.** Procédé de préparation d'un composé de formule Ia selon la revendication 2, lequel procédé comprend la réaction d'un composé de formule IV

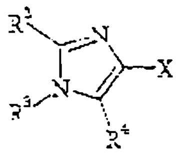 (IV)

avec un composé de formule V

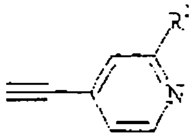 (V)

afin d'obtenir un composé de formule Ia ; où $R^1$, $R^2$, $R^3$ et $R^4$ sont tels que définis dans la revendication 1 et X est un halogène, et si souhaité, la conversion des composés obtenus en sels d'addition à un acide pharmaceutiquement acceptables.

**13.** Procédé de préparation d'un composé de formule Ia selon la revendication 2, lequel procédé comprend la réaction d'un composé de formule Ic

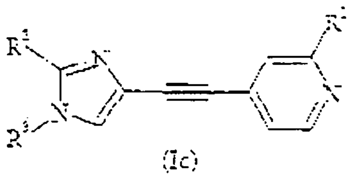

(Ic)

avec un composé de formule VI

$R^4$-X (VI)

afin d'obtenir un composé de formule Ia ; où $R^1$, $R^2$, $R^3$ et $R^4$ sont tels que définis dans la revendication 1 et X est un halogène, et si souhaité, la conversion des composés obtenus en sels d'addition à un acide pharmaceutiquement acceptables.

**14.** Procédé de préparation d'un composé de formule Ib selon la revendication 3, lequel procédé comprend la réaction d'un composé de formule XXVI

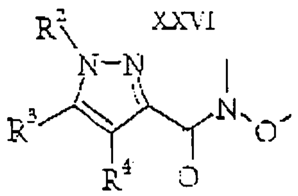

avec un composé de formule XXVII

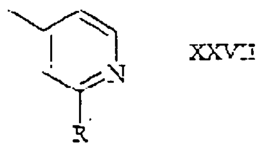

afin d'obtenir un composé de formule XXVIII

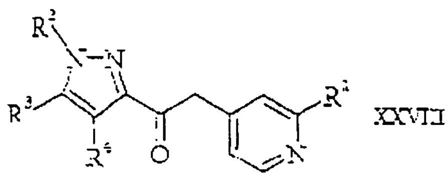

et de convertir le composé de formule XXVIII en composé de formule Ib ; où $R^1$, $R^2$, $R^3$ et $R^4$ sont tels que définis dans la revendication 1, et si souhaité la conversion des composés obtenus en sels d'addition à un acide pharmaceutiquement acceptables.

**15.** Médicament contenant un ou plusieurs composés tels que définis dans l'une quelconque des revendications 1 à 10 et des excipients pharmaceutiquement acceptables pour le traitement et la prévention des troubles médiés par les récepteurs mGluR5.

**16.** Médicament selon la revendication 15 pour le traitement et la prévention des troubles neurologiques aigus et/ou chroniques, en particulier de l'anxiété, ou pour le traitement de la douleur chronique et aiguë, la protection contre une lésion hépatique ou une insuffisance hépatique qu'elle soit induite par un médicament ou une maladie, l'incontinence urinaire, l'obésité, le syndrome du X fragile ou l'autisme.

**17.** Composé selon l'une quelconque des revendications 1 à 10 ainsi que son sel pharmaceutiquement acceptable pour une utilisation dans le traitement ou la prévention de maladies.

**18.** Utilisation d'un composé selon l'une quelconque des revendications 1 à 10 ainsi que son sel pharmaceutiquement acceptable pour la fabrication de médicaments destinés au traitement et à la prévention des troubles neurologiques aigus et chronique, des troubles cognitifs, des déficits de mémoire, de la douleur aiguë et chronique, de l'épilepsie, de la schizophrénie, de l'anxiété, des processus dégénératifs aigus, traumatiques ou chroniques du système nerveux, de la maladie d'Alzheimer, de la démence sénile, de la chorée de Huntington, de la sclérose amyotrophique latérale, de la sclérose en plaques, de la démence provoquée par le SIDA, des lésions oculaires, de la rétinopathie, de parkinsonisme idiopathique, du parkinsonisme provoqué par des médicaments, des spasmes musculaires, des convulsions, de la migraine, de l'incontinence urinaire, de l'addiction à l'éthanol, de l'addiction à la nicotine, des psychoses, de l'addiction aux opiacés, de l'anxiété, des vomissements, des dyskinésies, de la dépression, des restrictions de la fonction cérébrale provoquées par des opérations de pontage, d'un défaut d'apport de sang au cerveau, des lésions de la moelle épinière, des lésions crâniennes, d'une hypoxie provoquée par la grossesse, d'un arrêt cardiaque et d'une hypoglycémie, d'une lésion hépatique ou d'une insuffisance hépatique qu'elle soit induite par un médicament ou une maladie, de l'obésité, du syndrome du X fragile ou de l'autisme, et de l'incontinence urinaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0246166 A
- WO 0116121 A
- WO 2004007444 A
- WO 9613492 A
- EP 318704 A

### Non-patent literature cited in the description


- *Expert Opin. Ther. Patents,* 2002, vol. 12 (12
- **STORTO, MARIANNA ; BATTAGLIA, GIUSEPPE ; GRADINI, ROBERTO ; BRUNO, VALERIA ; NICOLETTI, FERDINANDO ; VAIRETTI, MARIAPIA.** Mouse hepatocytes lacking mGlu5 metabotropic glutamate receptors are less sensitive to hypoxic damage. *European Journal of Pharmacology,* 2004, vol. 497 (1), 25-27
- **STORTO, MARIANNA ; NGOMBA, RICHARD TEKE ; BATTAGLIA, GIUSEPPE ; FREITAS, ISABEL ; GRIFFINI, PATRIZIA ; RICHELMI, PLINIO ; NICOLETTI, FERDINANDO ; VAIRETTI, MARIAPIA.** Selective blockade of mGlu5 metabotropic glutamate receptors is protective against acetaminophen hepatotoxicity in mice. *Journal of Hepatology,* 2003, vol. 38 (2), 179-187
- **STORTO, MARIANNA ; DE GRAZIA, UGO ; KNOPFEL, THOMAS ; CANONICO, PIER LUIGI ; COPANI, AGATA ; RICHELMI, PLINIO ; NICOLETTI, FERDINANDO ; VAIRETTI, MARIAPIA.** Selective blockade of mGlu5 metabotropic glutamate receptors protects rat hepatocytes against hypoxic damage. *Hepatology,* 2000, vol. 31 (3), 649-655
- **SCHLAEGER ; CHRISTENSEN.** *Cytotechnology,* 1998, vol. 15, 1-13
- **PORTER et al.** *Br. J. Pharmacol.,* 1999, vol. 128, 13-20
- **ROBINSON ; STANISLAWSKI ; MULHOLLAND.** *The Journal of Organic Chemistry,* 2001, vol. 66 (12), 4148-4152
- **QIU ; STEVENSON ; O'BEIRNE ; SILVERMAN.** *J. Med. Chem.,* 1999, vol. 42, 329-332